(19) Europäisches Patentamt
European Patent Office
Office européen des brevets

(11) Veröffentlichungsnummer : **0 430 885 A2**

# (12) EUROPÄISCHE PATENTANMELDUNG

(21) Anmeldenummer : **90810907.7**

(22) Anmeldetag : **22.11.90**

(51) Int. Cl.[5] : **C07D 401/12,** C07D 403/12, A61K 31/505, A01N 43/54

(30) Priorität : **01.12.89 CH 4297/89**

(43) Veröffentlichungstag der Anmeldung : **05.06.91 Patentblatt 91/23**

(84) Benannte Vertragsstaaten : **AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Molleyres, Louis-Pierre, Dr.**
**Walter-Fürst-Strasse 5**
**CH-4102 Binningen (CH)**

(54) **Anthelminthika.**

(57) Es werden neue Anthelminthika beschrieben, die als Wirkstoff eine Verbindung der Formel I

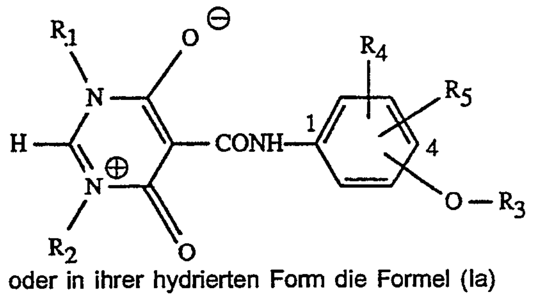

(I)

oder in ihrer hydrierten Form die Formel (Ia)

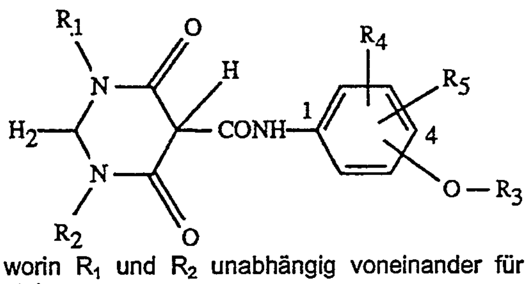

(Ia)

worin $R_1$ und $R_2$ unabhängig voneinander für $C_1$-$C_6$-Alkyl, Allyl, $C_3$-$C_6$-Cycloalkyl, Benzyl oder Phenyl stehen ;
$R_3$ einen unsubstituierten oder substituierten heteroaromatischen 6-gliederigen Ring, welcher 1, 2 oder 3 Stickstoffatome enthält oder einen benzokondensierten, unsubstituierten oder substituierten heteroaromatischen 6-gliederigen Ring, welcher 1 bis 3 Stickstoffatome enthält, und
$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl bedeuten, unter Einschluss deren tautomeren Formen und physiologisch verträglichen Salze. Es wird auch die Herstellung, Formulierung und Verwendung dieser Wirkstoffe beschrieben.

EP 0 430 885 A2

## ANTHELMINTHIKA

Die vorliegende Erfindung betrifft neue 1,3-disubstituierte Sechsringheterocyclyl-oxyphenylcarbamoyl-4,6-pyrimidindion-derivate der nachstehenden Formel I und deren hydrierte Analoga der nachstehenden Formel Ia mit anthelminthischer Wirksamkeit; besagte Substanzen zur Verwendung in einem Verfahren zur Bekämpfung von Helminthen; Anthelminthische Mittel die diese Substanzen als Wirkstoffe enthalten; die Herstellung der Wirkstoffe und Mittel; sowie die Verwendung der Wirkstoffe bzw. Mittel zur Bekämpfung von Helminthen, insbesondere von Nematoden, Cestoden und Trematoden in Haus- und Nutztieren aus der Klasse der Säugetiere.

Die erfindungsgemässen Verbindungen haben die Formel I

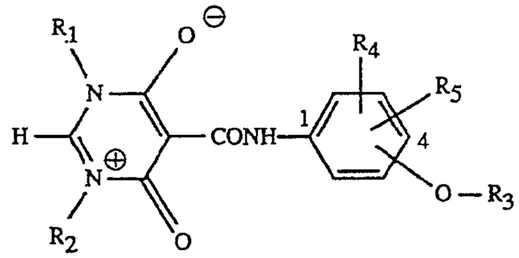

(I)

oder in ihrer hydrierten Form die Formel (Ia)

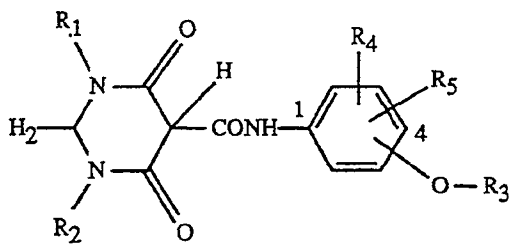

(Ia)

worin $R_1$ und $R_2$ unabhängig voneinander für $C_1$-$C_6$-Alkyl, Allyl, $C_3$-$C_6$-Cycloalkyl, Benzyl oder Phenyl stehen; $R_3$ einen unsubstituierten oder substituierten heteroaromatischen 6-gliederigen Ring, welcher 1, 2 oder 3 Stickstoffatome enthält oder einen benzokondensierten, unsubstituierten oder substituierten heteroaromatischen 6-gliederigen Ring, welcher 1 bis 3 Stickstoffatome enthält, und

$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl bedeuten, unter Einschluss deren tautomeren Formen und physiologisch verträglichen Salze.

In Formel I ist nur eine der zahlreichen möglichen Betainstrukturen wiedergegeben, weitere isoelektronische Strukturformeln sind z.B.:

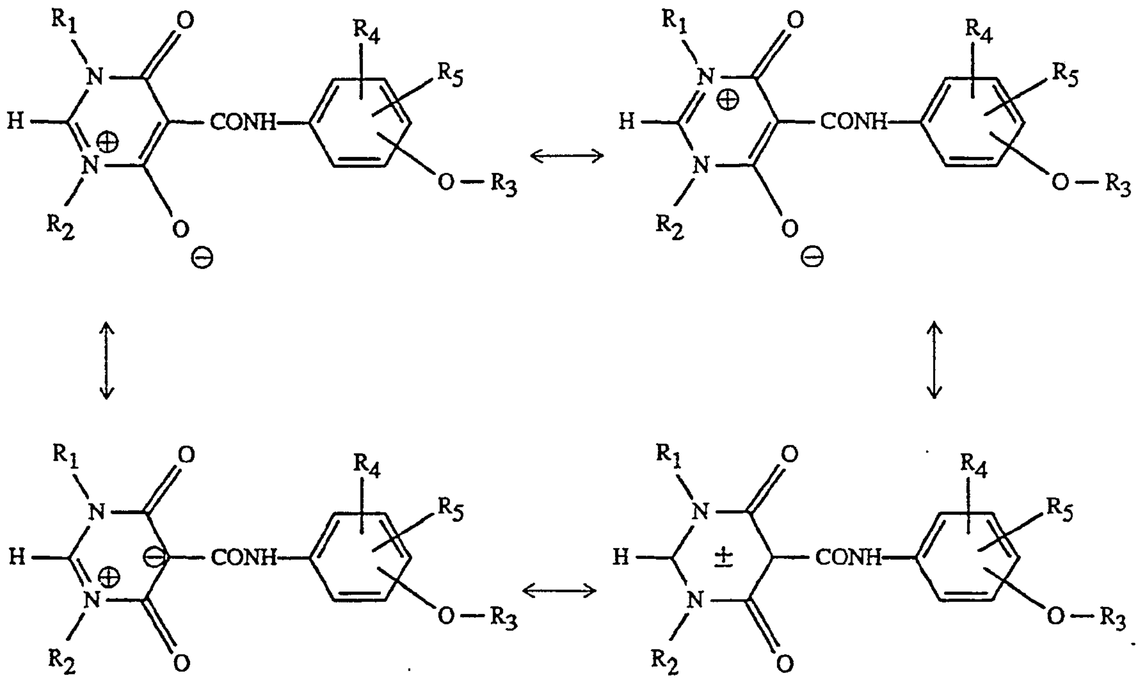

oder summarisch :

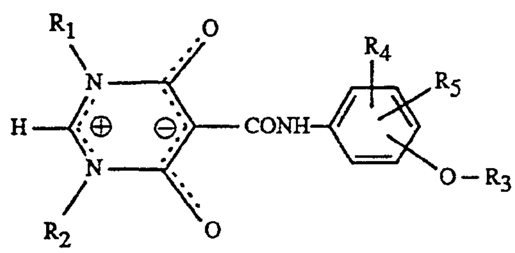

Die Verbindungen der Formel Ia können z.B. in folgenden tautomeren Formen vorliegen :

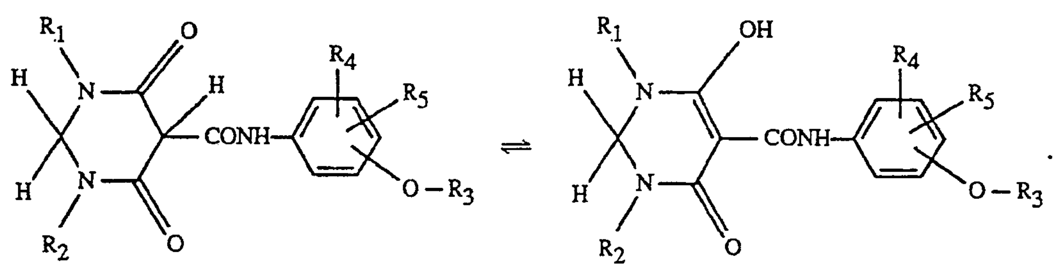

Die Verbindungen der Formeln I und Ia sind in allen Formen Bestandteil der vorliegenden Erfindung.

Unter dem Begriff Alkyl selbst oder als Bestandteil eines anderen Substituenten sind im Rahmen vorliegender Erfindung je nach Anzahl der angegebenen Kohlenstoffatome folgende geradkettige und verzweigte Gruppen zu verstehen, z.B. : Methyl, Ethyl, n-Propyl, iso-Propyl, n-Butyl, sek.-Butyl, tert.Butyl, Isobutyl, etc.. Haloalkyl selbst oder als Bestandteile für Haloalkoxy steht für einen einfach bis perhalogenierten Alkylsubstituenten, wie z.B. für $CH_2Cl$, $CHCl_2$, $CCl_3$, $CH_2F$, $CHF_2$, $CF_3$, $CH_2Br$, $CHBr_2$, $CBr_3$, $CH_2J$, $CJ_3$, CHClF, CHBrCl, CFBrCl, $C_2F_5$, $CH_2CH_2Cl$, $CHClCH_3$, $C_2Cl_5$, $CHFCHCl_2$, etc., vorzugsweise für $CF_3$. Unter Halogen soll hier und im folgenden Fluor, Chlor, Brom oder Jod, vorzugsweise Fluor, Chlor oder Brom, vor allem jedoch Chlor verstanden werden.

Cycloalkyl selbst oder als Bestandteil eines Substituenten steht je nach Anzahl der angegebenen Kohlenstoffatome für Cyclopropyl, Cyclobutyl, Cyclopentyl, Cyclohexyl, etc. Cyanoalkyl steht für eine Alkylgruppe, worin ein Wasserstoffatom durch CN substituiert ist, vorzugsweise für eine Alkylgruppe, worin sich

die CN-Gruppe am terminalen Kohlenstoffatom befindet.

Unter den Begriff physiologisch verträgliche Salze von Verbindungen der Formel I und Ia fallen die Alkalimetall-, Ammonium- oder Aminsalze, wobei Natrium-, Kalium-, Ammonium- oder Alkylaminsalze, insbesondere Triethylaminsalze, bevorzugt sind. Es sind darunter aber auch die Additionssalze anorganischer und organischer Säuren zu verstehen, die durch Anlagerung einer equivalenten Menge einer salzbildenden Säure an das Basismolekül entstehen.

Beispiele salzbildender Säuren sind anorganische Säuren : Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie z.B. Essigsäure, Trifluoressigsäure, Trichloressigsäure, Propionsäure, Glykolsäure, Thiocyansäure, Milchsäure, Bernsteinsäure, Zitronensäure, Ameisensäure, Benzolsulfonsäure, p-Toluolsulfonsäure, Methansulfonsäure, Salicylsäure, p-Aminosalicylsäure, Phthalsäure, 2-Phenoxybenzoesäure oder 2-Acetoxybenzoesäure.

Als Substituenten $R_3$ kommen beispielsweise unsubstituierte oder substituierte Vertreter der Gruppe Pyridin, Pyrimidinyl, Triazinyl, Pyrazinyl, Pyridazinyl, Chinolyl, Isochinolyl, Phthalazinyl, Chinoxalyl, Chinazolinyl, Cinnolinyl und Benzotriazinyl in Betracht.

Als Substituenten der vorgenannten Ringe und Ringsysteme sind $C_1$-$C_4$-Alkyl, Halogen-$C_1$-$C_4$-Alkyl, Alkoxyalkyl mit insgesamt 2 bis 4 Kohlenstoffatomen, $C_1$-$C_4$-Alkoxy, Halogen-$C_1$-$C_4$-Alkoxy, $C_1$-$C_4$-Alkylthio, Halogen-$C_1$-$C_4$-Alkylthio, $C_1$-$C_3$-Alkylamino, Di-($C_1$-$C_3$-alkyl)-amino, Allyl, Propargyl, Halogen, Nitro, Cyan, $C_3$-$C_6$-Cyclo- alkyl oder Phenyl zu nennen.

Als Substituent $R_3$ kommen demzufolge z.B. folgende cyclische Reste in Betracht :

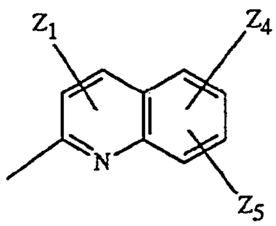
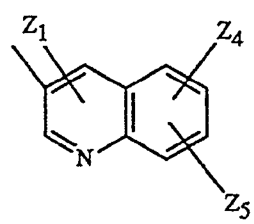
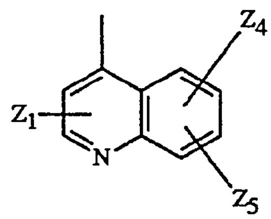
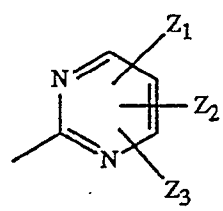

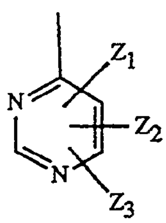
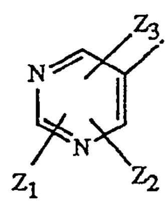
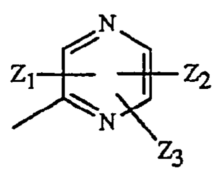
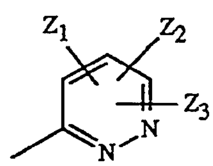

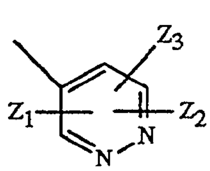
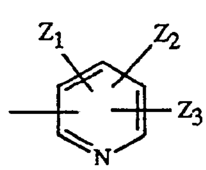

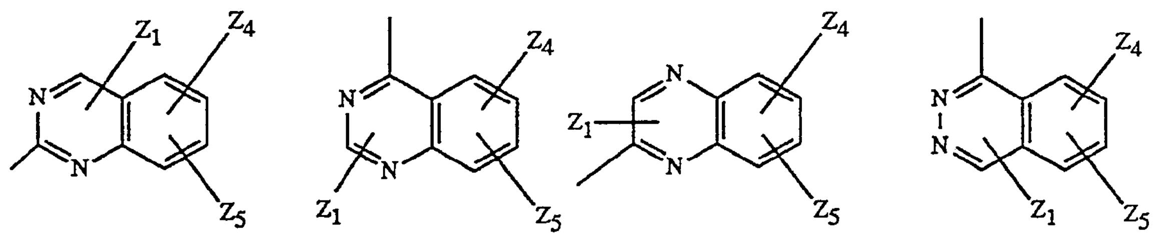

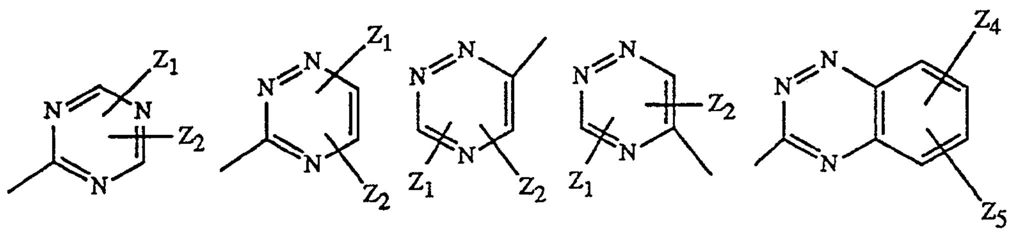

In den vorgenannten Resten stehen $Z_1$, $Z_2$, $Z_3$, $Z_4$ und $Z_5$ unabhängig voneinander für Wasserstoff, $C_1$-$C_4$-Alkyl, Halogen-$C_1$-$C_4$-Alkyl, Alkoxyalkyl mit insgesamt 2 bis 4 Kohlenstoffatomen, $C_1$-$C_4$-Alkoxy, Halogen-$C_1$-$C_4$-Alkoxy, $C_1$-$C_4$-Alkylthio, Halogen-$C_1$-$C_4$-Alkylthio, $C_1$-$C_3$-Alkylamino, Di-($C_1$-$C_3$-alkyl)-amino, Allyl, Propargyl, Halogen, Nitro, Cyan, $C_3$-$C_6$-Cycloalkyl oder Phenyl, wobei $Z_1$, $Z_2$ und $Z_3$ am heteroaromatischen, $Z_4$ und $Z_5$ am ankondensierten homoaromatischen Ring stehen. Besonders erwähnenswerte Reste $Z_1$, $Z_2$, $Z_3$, $Z_4$ und $Z_5$ sind Halogen, vorzugsweise Chlor, sowie Trifluormethyl und Methylthio.

Beispiele für $C_1$-$C_4$-Alkoxy sind Methoxy, Aethoxy, n-Propoxy, Isopropoxy, für $C_1$-$C_4$-Alkylthio Methylthio und n-Propylthio, für Halogen-$C_1$-$C_4$-Alkoxy Chlormethoxy, Fluormethoxy, 2-Chloräthoxy, 2,2-Dichloräthoxy, 3-Fluor-n-propoxy, und 2,2,2-Trifluoräthoxy, und für Halogen-$C_1$-$C_4$-Alkylthio Fluormethylthio, Chlormethylthio, 1,2-Dichloräthylthio und 2,2-Difluormethylthio.

Unter einem Di-($C_1$-$C_3$-Alkyl)-aminorest ist im Rahmen der vorliegenden Erfindung eine Aminogruppe zu verstehen, in welcher die beiden Wasserstoffatome durch zwei gleiche oder verschiedene Alkylgruppen mit 1 bis 3 Kohlenstoffatomen ersetzt sind. Bevorzugt ist die Dimethylaminogruppe.

Die Verbindungen der Formel I und Ia liegen bei Raumtemperatur überwie- gend als stabile Feststoffe vor, die einen Schmelzpunkt von ca. 100° bis ca. 300°C aufweisen. Sie besitzen sehr wertvolle anthelmintische Eigenschaften und lassen sich zur kurativen und präventiven Bekämpfung einer Reihe von Wurmerkrankungen bei Warmblütern, insbesondere bei Haus- und Nutztieren, vor allem aus der Klasse der Säugetiere einsetzen.

Nennenswert sind Verbindungen der Formel I, worin $R_3$, $R_4$ und $R_5$ die unter Formel I angegebenen Bedeutungen haben und $R_1$ und $R_2$ unabhängig voneinander für $C_1$-$C_6$-Alkyl, Allyl, $C_3$-$C_6$-Cycloalkyl oder Phenyl stehen.

Von besonderem Interesse sind Verbindungen der Formel I und Ia, in denen $R_1$ und $R_2$ unabhängig voneinander Methyl, Ethyl oder Allyl bedeuten, und insbesondere solche, in denen $R_1$ und $R_2$ unabhängig voneinander für Methyl oder Phenyl, vor allem für Methyl stehen.

Interessant sind auch die Vertreter im Umfang der Formel I und Ia worin $R_3$ ein unsubstituiertes oder substituiertes Pyridin, Pyrimidin oder Triazin bedeutet, wobei als Substituenten Methyl, Halomethyl insbesondere $CF_3$, Halogen insbesondere Chlor, Methylthio und Cyclopropyl in Frage kommen.

Eine interessante Gruppe stellen Verbindungen der Formel I und Ia dar, in denen $R_1$ und $R_2$ unabhängig voneinander für $C_1$-$C_4$-Alkyl, Allyl oder Cyclopropyl stehen ; $R_3$ einen unsubstituierten oder ein- bis dreifach durch Substituenten der Gruppe $C_1$-$C_4$-Alkyl, vorzugsweise Methyl, Isopropyl und tert.-Butyl, Methoxy, Methylamino, Dimethylamino, Methylthio, Trifluormethyl, Halogen, vorzugsweise Chlor, oder Phenyl substituierten Pyridyl, Pyrazinyl-, Pyridazinyl-, Triazinyl-, Pyrimidinyl-, Chinoxalinyl-, Chinolyl- oder Chinazolinylrest, $R_4$ Wasserstoff, $C_1$-$C_4$-Alkyl, vorzugsweise Methyl oder Isopropyl, oder Methoxy, und $R_5$ Wasserstoff oder Methyl bedeuten.

Besonders hervorzuheben sind ferner Verbindungen der Formel I und Ia, in denen das Strukturelement —$OR_3$ am Phenylring in meta- oder insbesondere in para- Stellung, vorzugsweise in para-Stellung, zum Carbamoylrest steht.

Eine besonders erwähnenswerte Gruppe bilden Verbindungen der Formel I, in denen $R_1$ und $R_2$ Methyl, $R_3$ einen unsubstituierten oder ein- oder zweifach durch Substituenten der Gruppe Methyl, Isopropyl, Methoxy oder Chlor substituierten Pyridyl, Pyrazinyl-, Pyrimidinyl-, Pyridazinyl-, Chinoxalinyl- oder Chinolylrest, und $R_4$ und $R_5$ Wasserstoff bedeuten.

Von herausragendem Interesse sind jedoch die nachfolgenden Gruppen von Verbindungen der Formeln

I und Ia, deren Bedeutung hinsichtlich ihrer Wirksamkeit von a) nach f) zunimmt.

a) Verbindungen in denen $R_1$ $C_1$-$C_4$-Alkyl, Allyl oder $C_3$-$C_6$-Cycloalkyl, $R_2$ $C_1$-$C_4$-Alkyl, $R_3$ einen unsubstituierten oder ein- bis dreifach durch Substituenten der Gruppe $C_1$-$C_4$-Alkyl, Halogen-$C_1$-$C_4$-Alkyl, $C_1$-$C_4$-Alkoxy, Halogen-$C_1$-$C_4$-Alkoxy, $C_1$-$C_4$-Alkylthio, $C_1$-$C_3$-Alkylamino, Di-($C_1$-$C_3$-alkyl)-amino, Allyl, Halogen, $C_3$-$C_6$-Cycloalkyl oder Phenyl substituierten, über ein Kohlenstoffatom gebundenen Pyridyl, Pyrazinyl-, Pyrimidinyl-, Pyridazinyl-, Triazinyl-, Chinoxalinyl- oder Chinolinylrest, $R_4$ Wasserstoff, $C_1$-$C_4$-Alkyl oder $C_1$-$C_4$-Alkoxy, und $R_5$ Wasserstoff bedeuten ;

b) Verbindungen in denen $R_1$ $C_1$-$C_2$-Alkyl, Allyl oder Cyclopropyl, $R_2$ $C_1$-$C_2$-Alkyl, $R_3$ einen unsubstituierten oder ein- bis dreifach durch Substituenten der Gruppe $C_1$-$C_4$-Alkyl, Halogen-$C_1$-$C_4$-Alkyl, $C_1$-$C_4$-Alkoxy, $C_1$-$C_4$-Alkylthio, Di-($C_1$-$C_3$-alkyl)-amino, Halogen oder Phenyl substituierten, über ein Kohlenstoffatom gebundenen Pyridyl, Pyrazinyl-, Pyrimidinyl-, Pyridazinyl-, Triazinyl-, Chinoxalinyl-oder Chinolinylrest, $R_4$ Wasserstoff, $C_1$-$C_4$-Alkyl oder $C_1$-$C_4$-Alkoxy, und $R_5$ Wasserstoff oder $C_1$-$C_4$-Alkyl bedeuten ;

c) Verbindungen, in denen $R_1$ $C_1$-$C_2$-Alkyl, Allyl oder Cyclopropyl, $R_2$ Methyl, $R_3$ einen unsubstituierten oder ein- bis dreifach durch Substituenten der Gruppe $C_1$-$C_4$-Alkyl, Trifluormethyl, Methoxy, Methylthio, Chlor oder Phenyl substituierten, über ein Kohlenstoffatom gebundenen Pyridyl, Pyrimidinyl-, Pyrazinyl- oder Pyridazinylrest, $R_4$ Wasserstoff, $C_1$-$C_4$-Alkyl oder $C_1$-$C_4$-Alkoxy, und $R_5$ Wasserstoff oder $C_1$-$C_4$-Alkyl bedeuten und der Molekülteil —$OR_3$ in meta- oder para-Stellung zum Stickstoffatom der Carbamoylgruppe steht ;

d) Verbindungen, in denen $R_1$ Methyl oder Allyl, $R_2$ Methyl, $R_3$ einen unsubstituierten oder ein- oder zweifach durch Substituenten der Gruppe $C_1$-$C_2$-Alkyl, Halogen-$C_1$-$C_2$-Alkyl, Halogen oder Phenyl substituierten, über ein Kohlenstoffatom gebundenen Pyridyl- oder Pyrimidinylring, $R_4$ Wasserstoff, $C_1$-$C_2$-Alkyl oder $C_1$-$C_2$-Alkoxy, $R_5$ Wasserstoff oder Methyl bedeuten und der Molekülteil —$OR_3$ in meta- oder para-Stellung zum Stickstoffatom der Carbamoylgruppe steht ;

e) Verbindungen, in denen $R_1$ Methyl, $R_2$ Methyl, $R_3$ einen ein- oder zweifach durch Substituenten der Gruppe Methyl, Trifluormethyl, Chlor oder Phenyl substituierten, über ein Kohlenstoffatom gebundenen Pyridyl- oder Pyrimidinylring, $R_4$ Wasserstoff, Methyl, Isopropyl, Methoxy oder Ethoxy, $R_5$ Wasserstoff oder Methyl bedeuten und der Molekülteil —$OR_3$ in meta- oder para-Stellung zum Stickstoffatom der Carbamoylgruppe steht ;

f) Verbindungen, in denen $R_1$ Methyl, $R_2$ Methyl, $R_3$ einen durch Trifluormethyl einfach oder durch Trifluormethyl und einen weiteren Substituenten der Gruppe Methyl, Trifluormethyl, Chlor und Phenyl insgesamt zweifach substituierten, über ein Kohlenstoffatom gebundenen Pyridyl- oder Pyrimidinylring, $R_4$ Wasserstoff, Methyl, Isopropyl oder Methoxy, $R_5$ Wasserstoff oder Methyl bedeuten und der Molekülteil —$OR_3$ in para-Stellung zum Stickstoffatom der Carbamoylgruppe steht.

Besonders bevorzugte Einzelvertreter der Formel I sind :

1,3-Dimethyl-5-[4-(4-trifluormethyl-6-chlorpyridyl-2-oxy)-phenylcarbamoyl]-4(6)-oxo-6(4)-oxido-(1H,5H)-pyrimidiniumbetain ;

1,3-Dimethyl-5-[4-(2-cyclopropyl-6-trifluormethylpyrimidyl-4-oxy)-phenylcarbamoyl]-4(6)-oxo-6(4)-oxido-(1H,5H)-pyrimidiniumbetain ;

1,3-Dimethyl-3-[3-(3-chlor-5-trifluormethylpyridyl-2-oxy)-4-chlorphenyl-carbamoyl]-4(6)-oxo-6(4)-oxido-(1H,5H)-pyrimidiniumbetain ;

1,3-Dimethyl-5-[4-(4-trifluormethylpyridyl-2-oxy)-phenylcarbamoyl]-4(6)-oxo-6(4)-oxido-(1H,5H)-pyrimidiniumbetain ;

1,3-Dimethyl-5-[4-(3-chlor-5-trifluormethylpyridyl-2-oxy)-phenylcarbamoyl]-4(6)-oxo-6(4)-oxido-(1H,5H)-pyrimidiniumbetain.

Besonders bevorzugte Vertreter der Formel Ia sind :

1,3-Dimethyl-5-[4-(4-trifluormethyl-6-chlorpyridyl-2-oxy)-phenylcarbamoyl]-4,6-(1H,3H,5H)-pyrimidindion ;

1,3-Dimethyl-5-[4-(2-cyclopropyl-6-trifluormethylpyrimidyl-4-oxy)-phenylcarbamoyl]-4,6-(1H,3H,5H)-pyrimidindion ;

1,3-Dimethyl-3-[3-(3-chlor-5-trifluormethylpyridyl-2-oxy)-4-chlorphenylcarbamoyl]-4,6-(1H,3H,5H)-pyrimidindion ;

1,3-Dimethyl-5-[4-(4-trifluormethylpyridyl-2-oxy)-phenylcarbamoyl]-4,6-(1H,3H,5H)-pyrimidindion ;

1,3-Dimethyl-5-[4-(3-chlor-5-trifluormethylpyridyl-2-oxy)-phenylcarbamoyl]-4,6-(1H,3H,5H)-pyrimidindion.

Die Verbindungen der Formeln I und Ia werden erfindungsgemäss dadurch hergestellt, dass man eine Verbindung der Formel II,

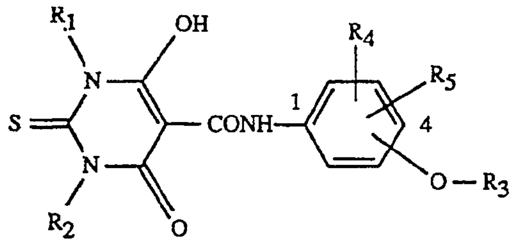

(II)

worin $R_1$, $R_2$ und $R_3$ die unter Formel I und Ia angegebenen Bedeutungen hat entschwefelt. Die Entschwefelung kann z.B. durch Hydrierung, vorzugsweise durch katalytische Hydrierung, vorgenommen werden. In einer bevorzugten Ausführungsform wird die Verbindung der Formel II in einem reaktionsinerten Lösungsmittel oder Lösungsmittelgemisch bei Temperaturen im Bereich von 20 bis 180°C, vorzugsweise 60 bis 120°C, insbesondere bei Rückflusstemperatur mit Trialkylzinnhydrid, Trialkylzinnhalogenid, Trialkylgermaniumhydrid, Trialkylgermaniumhalogenid, Alkylquecksilberhydrid oder Alkylquecksilberhalogenid hydriert, wobei bei der Verwendung eines Halogenids zusätzlich in Gegenwart von $NaBH_4$ gearbeitet wird. Alkyl bedeutet in diesem Zusammenhang vorzugsweise $C_1$-$C_6$-Alkyl, insbesondere $C_2$-$C_4$-Alkyl, Halogenid bedeutet insbesondere Chlorid oder Bromid. Besonders geeignet sind die Chloride. Als weiteres geeignetes Hydrierungsmittel ist Tris(trimethylsilyl)silan zu nennen.

Die Hydride und Halogenide werden in mindestens äquimolarer Menge, bezogen auf die Ausgangsverbindung der Formel II, eingesetzt. $NaBH_4$ kann ebenfalls äquimolar zugesetzt werden.

Man kann zusätzlich in Anwesenheit eines Radikalinitiators arbeiten, wobei dieser in katalytischen Mengen zugesetzt werden kann. Geeignete Radikalinitiatoren sind z.B. Azoisobutyronitril (AiBN), Peroxide wie Benzoylperoxid, aber auch UV-Licht oder Wärme. Als geeignete reaktionsinerte Lösungsmittel, allein oder im Gemisch untereinander, kommen z.B. in Frage : Aliphatische und aromatische Kohlenwasserstoffe, wie z.B. Pentan, Hexan, Petrolether, Ligroin, Benzol, Toluol, Xylole, usw. ; Ether und etherartige Substanzen wie Tetrahydrofuran, Anisol, Dioxan, usw. ; halogenierte Kohlenwasserstoffe wie Tetrachlorkohlenstoff, Tetrachlorethylen, Chlorbenzol, etc..

Die Verbindungen I und Ia fallen im allgemeinen nebeneinander an, wobei man durch geeignete Wahl des Lösungsmittels das System so wählen kann, dass z.B. die schwerlöslichen Betaine der Formel I ausfallen und aus dem Reaktionsgemisch z.B. durch Filtration, entfernt werden können, während die löslicheren Verbindungen der Formel Ia aus der Lösung, z.B. durch Einengen oder ausfällen, erhältlich sind. Man kann aber auch ein Gemisch von I und Ia isolieren und anschliessend, z.B. auf Grund ihres unterschiedlichen Lösungsverhaltens, eine Trennung vornehmen, z.B. durch fraktionierte Kristallisation oder durch Säulenchromatographie. Die geschickte Wahl der Lösungsmittel führt aber u.U. auch zu reinen Produkten, d.h. entweder vollständig zu I oder zu Ia. Die Verbindungen der Formel I lassen sich nach üblichen Methoden durch Hydrierung, z.B. mit Raney-Nickel, $NaBH_4$, Tributylzinnhydrid, etc., in die Verbindungen der Formel Ia überführen.

Wie bereits eingangs erwähnt, können die Verbindungen der Formel I und Ia auch als Addukte mit Basen oder Säuren vorliegen.

Während Säureadditionssalze bereits eingangs eingehend beschrieben wurden, sind hier noch die anorganischen und organischen Basen zu erwähnen, die als Adduktbildner in Frage kommen. Dies sind z.B. vorzugsweise tertiäre Amine wie Trialkylamine (z.B. Trimethylamin, Triethylamin oder Tripropylamin), Pyridin und Pyridinbasen (z.B. 4-Dimethylaminopyridin, oder 4-Pyrrolidylaminopyridin), Picoline und Lutidine sowie Oxide, Hydroxide, Carbonate und Hydrogencarbonate von Alkali- und Erdalkalimetallen (z.B. CaO, BaO, NaOH, KOH, $Ca(OH)_2$, $KHCO_3$, $NaHCO_3$, $K_2CO_3$ oder $Na_2CO_3$), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriummethylat. Die Base wird vorteilhafterweise in bezug auf die Reaktanden in 10 bis 100% der äquimolaren Menge zugesetzt.

In manchen Fällen kann es von Vorteil sein, die Reaktion unter Schutzgasatmosphäre durchzuführen. Geeignete Schutzgase sind z.B. Stickstoff, Helium oder Argon. Die Umsetzung von II mit Trialkylzinnhydrid, vor allem in Gegenwart eines Radikalstarters, ist besonders bevorzugt.

Die Thiobarbiturate der Formel II sind z.B. aus den Europäischen Offenlegungsschriften EP-191,474, EP-135,155 und EP-167,491 oder den korrespondierenden Britischen Offenlegungsschriften GB-2,174,388 B, GB-2,145,087 B und GB-2,163,423 B bekannt oder können analog zu den dort beschriebenen Vertretern hergestellt werden.

Die erfindungsgemässen Wirkstoffe der Formel I und Ia können in unterschiedlichen tautomeren Formen vorliegen, nämlich in der Keto- oder Enolform oder in einem Gemisch aus Keto- und Enolform. Die vorliegende

Erfindung betrifft sowohl die einzelnen Tautomeren als auch deren Gemische, aber auch die Salze jeder dieser Formen und deren Herstellung.

Das beschriebene Herstellungsverfahren einschliesslich aller Varianten ist ein Bestandteil vorliegender Erfindung.

Wie allgemein bekannt, verursachen unter den bei Warmblütern vorkommenden Endoparasiten namentlich die Helminthen bei den von ihnen heimgesuchten Tieren grosse Schäden. Solche durch Helminthiasen verursachten Schäden können bei chronischem und vor allem bei epidemischem Auftreten der Wurmerkrankungen in Viehherden ein volkswirtschaftlich ins Gewicht fallendes Ausmass annehmen. Sie äussern sich bei den erkrankten Tieren unter anderem in Produktivitäts-Einbussen, geschwächter Widerstandskraft gegenüber weiteren Krankheiten und erhöhter Mortalität. Besonders gefährliche Wurmkrankheiten werden durch im Magen-Darmtrakt und anderen Organen parasitierende Helminthen hervorgerufen und können beispielsweise bei Wiederkäuern wie Rindern, Schafen und Ziegen sowie Pferden, Schweinen, Geflügel, Rotwild, Hunden und Katzen auftreten.

In der vorliegenden Beschreibung werden unter dem Begriff Helminthen insbesondere parasitische Würmer verstanden, die zu den Plathelminthes (Cestoden, Trematoden) und Nemathelminthes (Nematoden und Verwandte) gehören, also Bandwürmer, Saugwürmer und Rundwürmer des Gastrointestinal-Traktes und anderer Organe (z.B. Leber, Lunge, Niere, Lymphgefässe, Blut etc.).

Es ist deshalb eine vordringliche Aufgabe, therapeutische Mittel zu entwickeln, die sich zur Bekämpfung von Helminthen in allen ihren Entwicklungsstadien sowie zur Vorbeugung gegen den Befall durch diese Parasiten eignen.

Es sind zwar eine Reihe von Stoffen mit anthelmintischer Wirkung bekannt, die für die Bekämpfung der verschiedenen Helminthen-Species vorgeschlagen wurden. Diese vermögen jedoch nicht voll zu befriedigen, sei es, dass bei verträglicher Dosierung eine Ausschöpfung ihres Wirkungsspektrums nicht möglich ist, oder dass sie in therapeutisch wirksamen Dosen unerwünschte Nebenwirkungen oder Eigenschaften zeigen. In diesem Zusammenhang spielt auch die heute vermehrt auftretende Resistenz gegen bestimmte Stoffklassen eine immer bedeutendere Rolle. Das beispielsweise in der Literatur beschriebene "Albendazol" (British Pat. No. 1464326 ; Am. J. Vet. Res. 38, 1425-1426 (1977) ; Am. J. Vet. Res. 37, 1515-1516 (1976) ; Am. J. Vet. Res. 38, 807-808 (1977) ; Am. J. Vet. Res. 38, 1247-1248 (1977)) besitzt nur ein begrenztes anthelmintisches Wirkungsspektrum bei Wiederkäuern. Seine Wirkung gegen Benzimidazol-resistente Nematoden und adulte Leberegel ist völlig unzureichend, wobei vor allem die pathogen wichtigen unreifen Wanderformen der letzteren bei den für das Wirtstier verträglichen Dosierungen nicht angegriffen werden.

Es wurde überraschenderweise gefunden, dass die neuen Verbindungen der Formel I und Ia ein breites Wirkungsspektrum gegen im Tierorganismus, vor allem in Säugetieren, parasitierende Helminthen wie Nematoden, Cestoden und Trematoden besitzen, wobei ihre Wirkung sich vorzugsweise gegen Nematoden (Rundwürmer) richtet.

Als besonderes Merkmal der Verbindungen der Formel I und Ia ist ihre gegenüber Warmblütern überraschend hohe Verträglichkeit hervorzuheben, die sie gegenüber den bekannten Thiobarbitursäurederivaten überlegen macht. Ihre praktische Handhabung bei der Behandlung Wurmerkrankter Tiere wird dadurch ausserordentlich erleichtert, da sie auch in höheren Dosen von den medikierten Tieren symptomlos vertragen werden.

Die erfindungsgemässen neuen Wirkstoffe der Formel I und Ia eignen sich beispielsweise zur Bekämpfung parasitärer Nematoden der Ordnungen (nach K.I. Skrajabin)

Rhabditida
Ascaridida
Spirurida
Trichocephalida

oder zur Bekämpfung von Cestoden der Ordnungen (nach Wardle & McLeod)

Cyclophyllidae
Pseudophyllidae

oder zur Bekämpfung von Trematoden der Ordnung

Digenea

bei Haus- und Nutztieren wie Rindern, Schafen, Ziegen, Pferden, Schweinen, Rotwild, Katzen, Hunden und Geflügel. Sie können den Tieren sowohl als Einzeldosis als auch wiederholt verabreicht werden, wobei die einzelnen Gaben je nach Tierart vorzugsweise zwischen 1 und 20 mg pro kg Körpergewicht betragen. Durch eine protrahierte Verabreichung kann in manchen Fällen eine bessere Wirkung erzielt oder mit geringeren Gesamtdosen ausgekommen werden.

Die erfindungsgemässen Mittel werden hergestellt, indem die Wirkstoffe der Formel I oder Ia mit flüssigen und/oder festen Formulierungshilfsstoffen durch schrittweises Vermischen und/oder Vermahlen derart in

Kontakt gebracht werden, dass eine anwendungskonforme optimale Entfaltung der anthelmintischen Aktivität der Formulierung erzielt wird.

Die Formulierungsschritte können durch Kneten, Granulieren (Granulate) und gegebenenfalls Pressen (Pellets) ergänzt werden.

Als Formulierungshilfsstoffe dienen beispielsweise feste Trägerstoffe, Lösungsmittel und gegebenenfalls oberflächenaktive Stoffe (Tenside).

Zur Bereitung der erfindungsgemässen Mittel werden folgende Formulierungshilfsstoffe verwendet : Feste Trägerstoffe wie z.B. Kaolin, Talkum, Bentonit, Kochsalz, Calcium-phosphat, Kohlenhydrate, Cellulosepulver, Baumwollsaatmehl, Polyäthylenglykoläther, gegebenenfalls Bindemittel wie z.B. Gelatine, lösliche Cellulosederivate, gewünschtenfalls unter Zusatz von oberflächenaktiven Stoffen wie ionischen oder nicht-ionischen Dispersionsmitteln ; ferner natürliche Gesteinsmehle wie Calcit, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden.

Als Lösungsmittel kommen in Frage : Aromatische kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat; aliphatische Kohlenwasserstoffe wie z.B. Cyclohexan oder Paraffine, Alkohole und Glykole sowie deren Ether und Ester, wie z.B. Ethanol, Ethylenglykol, Ethylenglykolmonomethyl- oder -ethylether, Ketone wie z.B Cyclohexanon, stark polare Lösungsmittel wie z.B. N-Methyl-2-pyrrolidon, Dimethylsulfoxid oder Dimethylformamid, sowie gegebenenfalls epoxydierte Pflanzenöle wie z.B epoxydiertes Kokosnussöl oder Sojaöl und Wasser.

Als oberflächenaktive Verbindungen kommen je nach Art des zu formulierenden Wirkstoffes der Formel I oder Ia nicht-ionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufig werden sog. synthetische Tenside verwendet, insbesondere Fettsulfonate, Fettsulfate, sulfonierte Benzimidazolderivate oder Alkylarylsulfonate.

Die Fettsulfonate oder —sulfate liegen in der Regel als Alkali—, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes oder Phospholipide in Frage.

Als nicht-ionische Tenside kommen in erster Linie Polyglykoletherderivate von aliphatischen oder cycloaliphatischen Alkoholen, gesättigten oder ungesättigten Fettsäuren und Alkyphenolen in Frage, die 3 bis 30 Glykolethergruppen und 8 bis 20 Kohlenstoffatome im aliphatischen Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Ethylenglykoleinheiten.

Als Beispiele nicht-ionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyethylensorbitan-trioleat in

Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulieiungstechnik gebräuchlichen Tenside sind u.a. in folgenden Publikationen beschrieben:
"Mc Cutcheon's Detergents and Emulsifiers Annual",
MC Publishing Corp., Ridgewood New Jersey, 1980 ;
Sisley and Wood, "Encyclopedia of Surface Active Agents",
Chemical Publishing Co., Inc. New York, 1980.

Als Bindemittel für Tabletten und Boli kommen chemisch abgewandelte, in Wasser oder Alkohol lösliche, polymere Naturstoffe in Frage, wie Stärke-, Cellulose- oder Protein-derivate (z.B. Methylcellulose, Carboxymethylcellulose, Ethylhydroxyethylcellulose, Proteine wie Zein, Gelatine und dergleichen) sowie synthetische Polymere wie z.B. Polyvinylalkohol, Polyvinylpyrrolidon etc.. Ferner sind in Tabletten Füllstoffe (z.B. Stärke, mikrokristalline Cellulose, Zucker, Milchzucker etc.), Gleitmittel und Sprengmittel enthalten.

Liegen die anthelmintischen Mittel in Form von Futterkonzentraten vor, so dienen als Trägerstoffe z.B. Leistungsfutter, Futtergetreide oder Proteinkonzentrate. Solche Futterkonzentrate oder -mittel können ausser den Wirkstoffen noch Zusatzstoffe, Vitamine, Antibiotika, Chemotherapeutika, oder andere Pestizide, vornehmlich Bakteriostatika, Fungistatika, Coccidiostatika, oder auch Hormonpräparate, Stoffe mit anaboler Wirkung oder das Wachstum begünstigende, die Fleischqualität von Schlachttieren beeinflussende oder in anderer Weise für den Organismus nützliche Stoffe enthalten. Werden die Mittel oder die darin enthaltenen Wirkstoffe der Formel I direkt dem Futter oder den Viehtränken zugesetzt, so enthält das Fertigfutter oder die Fertigtränke die Wirkstoffe vorzugsweise in einer Konzentration von etwa 0,0005 bis 0,02 Gewichtsprozent (5-200 ppm).

Die Applikation der erfindungsgemässen Mittel an die zu behandelnden Tiere kann peroral, parenteral oder subcutan durchgeführt werden, wobei die Mittel in Form von Lösungen, Emulsionen, Suspensionen (Drenchs), Pulvern, Tabletten, Boli und Kapseln vorliegen.

Die erfindungsgemässen anthelmintischen Mittel enthalten in der Regel 0,1 bis 99 Gew.-%, insbesondere 0,1 bis 95 Gew.-% Wirkstoff der Formel I, Ia oder Gemische davon, 99,9 bis 1 Gew.-%, insbesondere 99,8 bis 5 Gew.-%, eines festen oder flüssigen Zusatzstoffes, darunter 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Derartige vom Endverbraucher verwendete anthelmintische Mittel sind ebenfalls ein Bestandteil der vorliegenden Erfindung.

In jedem der erfindungsgemässen Verfahren zur Schädlingsbekämpfung bzw. der erfindungsgemässen Schädlingsbekämpfungsmittel können die Wirkstoffe der Formel I und Ia in allen tautomeren Formen, deren Mischungen oder in Form ihrer Salze eingesetzt werden.

Die Erfindung schliesst auch ein Verfahren zum prophylaktischen Schutz von Tieren gegen parasitäre Helminthen ein, das dadurch gekennzeichnet ist, dass man die Wirkstoffe der Formel I und Ia bzw. die Wirkstofformulierungen als Zusatz zum Futter oder zu den Tränken oder auch in fester oder flüssiger Form oral, durch Injektion oder parenteral den Tieren appliziert.

Die nachfolgenden Beispiele dienen der Illustration der Erfindung, ohne sie einzuschränken.

## 1. Herstellungsbeispiele

### 1.1. Herstellung von 1,3-Dimethyl-5-[4-(4-trifluormethyl-6-chlor-pyridyl-2-oxy)-phenylcarbamoyl]-4(6)-oxo-6(4)-oxido-(1H,5H)-pyrimidiniumbetain

Eine Lösung von 8 g (16,5 mMol) 1,3-Dimethyl-5-[4-(4-trifluormethyl-6-chlor-pyridyl-2-oxy)-phenylcarbamoyl]-2-thiobarbitursäure, 14,3 g (49,2 mMol) Tributylzinnhydrid und 207 mg (1,26 mMol) Azoisobutyronitril in 350 ml Benzol wird unter Rückfluss und Stickstoffatmosphäre 45 Minuten erhitzt. Nach Abkühlen des Reaktionsgemisches wird das ausgefallene Produkt filtriert, mehrfach mit Hexan gewaschen und dann in Benzol suspendiert und heiss filtriert. Man erhält 1,7 g (23%) der Titelsubstanz. Smp. 295-297°C.

$^1$H-NMR (300 MHz, DMSO d6, TMS) : 11,06, s, NH ; 9,38, s, H-C(3) ; 7,70, s, 1H ; 7,69, d, J = 9,5, 2H ; 7,45, s, 1H ; 7,15, d, J = 9,5, 2H ; 3,38, s, 2 x $CH_3$.

1.2. Herstellung von 1,3-Dimethyl-5-[4-(4-trifluormethyl-6-chlor-pyridyl-2-oxy)-phenylcarbamoyl]-4,6-(1H,3H,5H)-pyrimidindion

Das gemäss Beispiel 1.1. gewonnene Filtrat wird eingeengt und das ausgefallene Produkt abfiltriert und mit Essigester umkristallisiert. Man erhält 4,1 g (55%) der Titelsubstanz. Smp. 174-175°C.

$^1$H-NMR (300 MHz, $CDCl_3$, TMS) : 18,22, s, OH ; 11,87, s, NH ; 7,55, d, J = 9,5, 2H ; 7,23, s, 1H ; 7,12, d, J = 9,5, 2H ; 7,00, s, 1H ; 4,49, s, $H_2$-C(3) ; 3,00, s, $CH_3$ ; 2,97, s, $CH_3$.

1.3. Herstellung von 1,3-Dimethyl-5-[4-(2-cyclopropyl-6-trifluormethylpyrimidyl-4-oxy)-phenylcarbamoyl]-4(6)-oxo-6(4)-oxido-(1H,5H)- pyrimidiniumbetain

Eine Lösung von 9,9 g (0,01 mMol) 1,3-Dimethyl-5-[4-(2-cyclopropyl-6-trifluormethyl-pyrimidyl-4-oxy)-phenylcarbamoyl]-2-thiobarbitursäure, 123 mg (0,75 mMol) Azoisobutyronitril und 8 ml (0,03 Mol) Tributylzinnhydrid werden 1 Stunde in 200 ml Benzol und unter Stickstoff unter Rückfluss erhitzt. Nach Abkühlen des Reaktionsgemisches wird das ausgefallene Produkt mit Benzol, dann mit Hexan mehrfach gewaschen und getrocknet. Man erhält 3,4 g (37%) der Titelsubstanz. Smp. 290-291°C.

$^1$H-NMR (300 MHz, DMSO d6, TMS) : 11,07, s, NH ; 9,38, s, H-C(3) ; 7,70, d, J = 7,5, 2H ; 7,58, s, 1H ; 7,18, d, J = 7,5, 2H ; 3,39, s, 2 x $CH_3$ ; 2,16-2,07, m, 1H ; 1,06-0,99, m, 2H ; 0,90-0,83, m, 2H.

1.4. Herstellung von 1,3-Dimethyl-5-[4-(2-cyclopropyl-6-trifluormethylpyrimidyl-4-oxy)-phenylcarbamoyl]-4,6-(1H,3H,5H)-pyrimidindion

a) Das Filtrat der obigen Reaktion nach 1.3. wurde eingeengt und der Rückstand aus Benzol umkristallisiert. Es werden 4,8 g (52%) des gewünschten Produktes isoliert.

$^1$H-NMR (300 MHz, $CDCl_3$, TMS) : 18,24, s, OH ; 11,90, s, NH ; 7,55, d, J = 9,5, 2H ; 7,09, d, J = 9,5, 2H ; 6,88, s, 1H ; 4,50, s, $H_2$-C(3) ; 3,01, s, $CH_3$ ; 2,96, s, $CH_3$ ; 2,24-2,12, m, 1H ; 1,07-0,98, m, 4H.

b) Eine Suspension von 461 mg (1 mMol) 1,3-Dimethyl-5-[4-(2-cyclopropyl-6-trifluormethyl-pyrimidyl-4-oxy)-phenylcarbamoyl]-4(6)-oxo-6(4)-oxido-(1H,5H)-pyrimdiniumbetain und 34 mg (1 mMol) $NaBH_4$ in Aethanol (10 ml) wurde unter Stickstoff 2 Stunden bei Raumtemperatur gerührt. Das auf 0°C abgekühlte Reaktionsgemisch wird mit 2 NHCl angesäuert, dann mit $NaHCO_3$ neutralisiert und mit Essigester extrahiert. Die organische Phase wird mit gesättigter $NaHCO_3$-Lösung und dann mit gesättigter NaCl-Lösung gewaschen, auf $MgSO_4$ getrocknet und eingeengt. Man erhält nach Kristallisation in Essigester, 337 mg (73%) der Titelsubstanz. Smp. 184-187°C.

1.5. Herstellung von 1,3-Dimethyl-5-[3-(3-chlor-5-trifluormethyl-pyridyl-2-oxy)-4-chlorphenylcarbamoyl]-4,6-(1H,3H,5H)-pyrimidindion

Eine Lösung von 17,5 g (34 mMol) 1,3-Dimethyl-3-[3-(3-chlor-5-trifluormethyl-pyridyl-2-oxy)-4-chlorphenylcarbamoyl]-2-thiobarbitursäure, 29,3 g (100 mMol) Tributylzinnhydrid und 490 mg (3 mMol) Azoisobutyronitril wird in 750 ml Toluol 7 Stunden unter Rückfluss gerührt, wobei man unter Stickstoffatmosphäre arbeitet. Das auf Raumtemperatur abgekühlte Gemisch wird eingeengt und das ausgefallene Produkt abfiltriert, mit Hexan gewaschen und am Hochvakuum getrocknet. Man erhält 13,68 g (83%) der Titelsubstanz. Smp. 175-185°C.

$^1$H-NMR (300 MHz, $CDCl_3$, TMS) : 18,21, s, OH ; 12,00, s, NH ; 8,26, d, J < 1, 1H ; 8,00, d, J < 1, 1H ; 7,62, d J < 1, 1H ; 7,41, d, J = 9, 1H ; 7,30, d x d, $J_1$ = 9, $J_2$ < 1, 1H ; 4,50, s, $H_2$-C(3) ; 3,01, s, $CH_3$ ; 2,95, s, $CH_3$.

Analog zu den beschriebenen Vorgehensweisen lassen sich auf die in den folgenden Tabellen genannten Vertreter der Formeln I und Ia herstellen.

Tabelle 1 : Pyrimidiniumbetain-Derivate der Formel

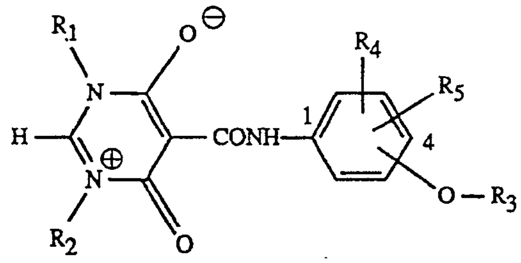

(I), worin $R_3$ für

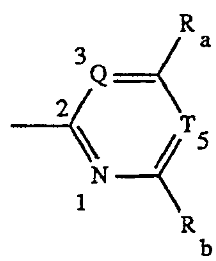

steht:

| Verb. Nr. | $R_1$ | $R_2$ | $R_4$ | $R_5$ | Position von $OR_3$ | Q | T | $R_a$ | $R_b$ | Smp. [°C] |
|---|---|---|---|---|---|---|---|---|---|---|
| 1.1 | $CH_3$ | $CH_3$ | H | H | 4 | C-Cl | C-$CF_3$ | H | H | 270-275° |
| 1.2 | $CH_3$ | $CH_3$ | H | H | 4 | CH | N | $CF_3$ | Cyclopropyl | 290-291° |
| 1.3 | $CH_3$ | $CH_3$ | H | H | 4 | CH | CH | $CF_3$ | H | 295-297° |
| 1.4 | $CH_3$ | $CH_3$ | H | H | 4 | CH | CH | $CF_3$ | Cl | 293-295° |
| 1.5 | $CH_3$ | $CH_3$ | H | 4-Cl | 3 | C-Cl | C-$CF_3$ | H | H | 304-305° |
| 1.6 | $CH_3$ | $CH_3$ | H | H | 4 | CH | N | Cl | H | |
| 1.7 | $CH_3$ | $CH_3$ | H | H | 4 | N | CH | $CH_3$ | $CH_3$ | |
| 1.8 | $CH_3$ | $CH_3$ | H | H | 4 | CH | N | $CH_3$ | $SCH_3$ | |
| 1.9 | $CH_3$ | $CH_3$ | H | H | 4 | CH | N | Cl | $C(CH_3)_2CH_2Cl$ | |
| 1.10 | $CH_3$ | $CH_3$ | H | H | 4 | CH | N | Cl | $CH_3$ | |
| 1.11 | $CH_3$ | $CH_3$ | H | H | 4 | CH | N | H | $SCH_3$ | |
| 1.12 | $CH_3$ | $CH_3$ | H | H | 4 | C-$CF_3$ | N | H | Cl | |
| 1.13 | $CH_3$ | $CH_3$ | H | H | 4 | N | C-$CH_3$ | H | H | |
| 1.14 | $CH_3$ | $CH_3$ | H | H | 4 | N | C-$CF_3$ | H | H | |
| 1.15 | $CH_3$ | $C_2H_5$ | H | H | 4 | CH | N | $CF_3$ | Cyclopropyl | 286-288° |
| 1.16 | $CH_3$ | $CH_2CH=CH_2$ | H | H | 4 | CH | N | $CF_3$ | Cyclopropyl | |
| 1.17 | $CH_3$ | $CH_3$ | 2-$OCH_3$ | H | 4 | CH | N | $CF_3$ | H | |

Fortsetzung von Tabelle 1:

| Verb. Nr. | $R_1$ | $R_2$ | $R_4$ | $R_5$ | Position von $OR_3$ | Q | T | $R_a$ | $R_b$ | Smp [°C] |
|---|---|---|---|---|---|---|---|---|---|---|
| 1.18 | $CH_3$ | $CH_3$ | 2-$CH_3$ | H | 4 | CH | N | H | $CF_3$ | |
| 1.19 | $CH_3$ | $CH_3$ | 3-Cl | H | 4 | C-Cl | C-$CF_3$ | H | H | |
| 1.20 | $CH_3$ | $CH_3$ | H | H | 4 | CH | C-$CF_3$ | H | H | 265-266° |
| 1.21 | $CH_3$ | $CH_3$ | 2-isopropyl | H | 4 | CH | C-$CF_3$ | H | H | |
| 1.22 | $CH_3$ | $CH_3$ | H | H | 3 | CH | C-$NO_2$ | H | H | |
| 1.23 | $CH_3$ | $CH_3$ | H | H | 4 | C-Cl | C-Cl | H | H | 320-322° |
| 1.24 | $CH_3$ | $C_2H_5$ | H | H | 4 | CH | CH | $CF_3$ | H | 278-280° |
| 1.25 | $CH_3$ | $C_2H_5$ | H | H | 4 | C-Cl | C-$CF_3$ | H | H | |
| 1.26 | $CH_3$ | $C_2H_5$ | H | H | 4 | CH | CH | $CF_3$ | Cl | |
| 1.27 | $CH_3$ | $CH_2CH=CH_2$ | H | H | 4 | CH | CH | $CF_3$ | H | 260-262° |
| 1.28 | $CH_3$ | $CH_2CH=CH_2$ | H | H | 4 | CH | CH | $CF_3$ | Cl | |
| 1.29 | $CH_3$ | $CH_2CH=CH_2$ | H | H | 4 | C-Cl | C-$CF_3$ | H | H | |
| 1.30 | $CH_3$ | $CH_3$ | 2-$CH_3$ | H | 4 | CH | CH | $CF_3$ | H | |
| 1.31 | $CH_3$ | $CH_3$ | 3-$OCH_3$ | H | 4 | CH | CH | $CF_3$ | H | |
| 1.32 | $C_2H_5$ | $C_2H_5$ | 2-Cl | 6-Cl | 4 | CH | CH | $CF_3$ | H | |

Fortsetzung von Tabelle 1:

| Verb. Nr. | $R_1$ | $R_2$ | $R_4$ | $R_5$ | Position von $OR_3$ | Q | T | $R_a$ | $R_b$ | Smp [°C] |
|---|---|---|---|---|---|---|---|---|---|---|
| 1.33 | $CH_3$ | $CH_3$ | 2-$CH_3$ | H | 4 | C-Cl | C-$CF_3$ | H | H | 261-263° |
| 1.34 | $CH_3$ | $CH_3$ | 2-F | H | 4 | C-Cl | C-$CF_3$ | H | H | 265-267° |
| 1.35 | $CH_3$ | $CH_3$ | 2-$CF_3$ | H | 4 | C-Cl | C-Cl | H | H | 295-296° |
| 1.36 | $CH_3$ | $CH_3$ | 2-$C_2H_5$ | 2-$C_2H_5$ | 4 | N | CH | H | H | |
| 1.37 | $CH_3$ | $CH_3$ | 2-$CH_3$ | 6-$CH_3$ | 4 | C-Cl | C-$CF_3$ | H | H | |
| 1.38 | $CH_3$ | $CH_3$ | 3-Cl | H | 4 | N | N | $OCH_3$ | $OCH_3$ | 281-283° |
| 1.39 | $CH_3$ | $CH_3$ | H | H | 4 | N | N | $OCH_3$ | $OCH_3$ | 280-282° |
| 1.40 | $CH_3$ | $CH_3$ | 2-$CH_3$ | 6-$CH_3$ | 4 | CH | C-$CF_3$ | H | H | |
| 1,41 | $CH_3$ | $CH_3$ | 2-$CH_3$ | 6-$CH_3$ | 4 | N | CH | H | H | 308-310° |
| 1.42 | $CH_3$ | $CH_3$ | H | H | 4 | C-$CH_3$ | C-Cl | $CH_3$ | N | |
| 1.43 | $CH_3$ | $CH_3$ | 2-$CH_3$ | 6-$CH_3$ | 4 | CH | C-Cl | H | N | |
| 1.44 | $CH_3$ | $CH_3$ | 2-Cl | 6-Cl | 4 | CH | CH | H | H | |
| 1.45 | $CH_3$ | $CH_3$ | 2-$CH_3$ | 6-$CH_3$ | 4 | C-Cl | C-Cl | H | H | |
| 1.46 | $CH_3$ | $CH_3$ | 3-Cl | 5-Cl | 4 | C-Cl | C-$CF_3$ | H | H | |
| 1.47 | $CH_3$ | $CH_3$ | H | H | 4 | C-Cl | C-$C_2Cl_5$ | H | H | |

Fortsetzung von Tabelle 1:

| Verb. Nr. | $R_1$ | $R_2$ | $R_4$ | $R_5$ | Position von $OR_3$ | Q | T | $R_a$ | $R_b$ | Smp [°C] |
|---|---|---|---|---|---|---|---|---|---|---|
| 1.48 | $CH_3$ | $CH_3$ | 3-Cl | H | 4 | C-Cl | $CCF_2CF_2Cl$ | H | H | |
| 1.49 | $CH_3$ | $CH_3$ | H | H | 4 | C-Cl | $CCF_2CF_2Cl$ | H | H | |
| 1.50 | $CH_3$ | $CH_3$ | 3-Cl | H | 4 | CH | CH | $CF_3$ | Cl | |
| 1.51 | $CH_3$ | $CH_3$ | 3-F | H | 4 | C-F | C-Cl | H | H | |
| 1.52 | $CH_3$ | $CH_3$ | 2-$C_3H_7$i | 6-$C_3H_7$i | 4 | C-Cl | $CF_3$ | H | H | |

Tabelle 2 : Pyrimidindion-Derivate der Formel

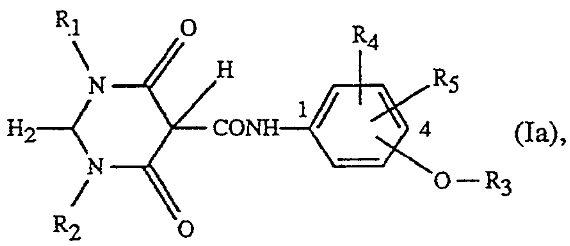 (Ia), worin $R_3$ für 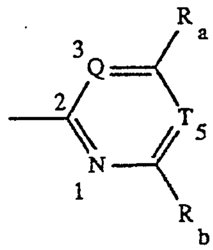 steht:

| Verb. Nr. | $R_1$ | $R_2$ | $R_4$ | $R_5$ | Position von $OR_3$ | Q | T | $R_a$ | $R_b$ | Smp. [°C] |
|---|---|---|---|---|---|---|---|---|---|---|
| 2.1 | $CH_3$ | $CH_3$ | H | H | 4 | C-Cl | C-$CF_3$ | H | H | 144-145° |
| 2.2 | $CH_3$ | $CH_3$ | H | H | 4 | CH | N | $CF_3$ | Cyclopropyl | 184-187° |
| 2.3 | $CH_3$ | $CH_3$ | H | H | 4 | CH | CH | $CF_3$ | H | 194-196° |
| 2.4 | $CH_3$ | $CH_3$ | H | H | 4 | CH | CH | $CF_3$ | Cl | 174-175° |
| 2.5 | $CH_3$ | $CH_3$ | H | 4-Cl | 3 | C-Cl | C-$CF_3$ | H | H | 199-200° |
| 2.6 | $CH_3$ | $CH_3$ | H | H | 4 | CH | N | Cl | H | |
| 2.7 | $CH_3$ | $CH_3$ | H | H | 4 | N | CH | $CH_3$ | $CH_3$ | |
| 2.8 | $CH_3$ | $CH_3$ | H | H | 4 | CH | N | $CH_3$ | $SCH_3$ | |
| 2.9 | $CH_3$ | $CH_3$ | H | H | 4 | CH | N | Cl | $C(CH_3)_2CH_2Cl$ | |
| 2.10 | $CH_3$ | $CH_3$ | H | H | 4 | CH | N | Cl | $CH_3$ | |
| 2.11 | $CH_3$ | $CH_3$ | H | H | 4 | CH | N | H | $SCH_3$ | |
| 2.12 | $CH_3$ | $CH_3$ | H | H | 4 | C-$CF_3$ | N | H | Cl | |
| 2.13 | $CH_3$ | $CH_3$ | H | H | 4 | N | C-$CH_3$ | H | H | |
| 2.14 | $CH_3$ | $CH_3$ | H | H | 4 | N | C-$CF_3$ | H | H | |
| 2.15 | $CH_3$ | $C_2H_5$ | H | H | 4 | CH | N | $CF_3$ | Cyclopropyl | 128-129° |
| 2.16 | $CH_3$ | $CH_2CH=CH_2$ | H | H | 4 | CH | N | $CF_3$ | Cyclopropyl | 165-166° |
| 2.17 | $CH_3$ | $CH_3$ | 2-$OCH_3$ | H | 4 | CH | N | $CF_3$ | H | |

Fortsetzung von Tabelle 2:

| Verb. Nr. | $R_1$ | $R_2$ | $R_4$ | $R_5$ | Position von $OR_3$ | Q | T | $R_a$ | $R_b$ | Smp [°C] |
|---|---|---|---|---|---|---|---|---|---|---|
| 2.18 | $CH_3$ | $CH_3$ | 2-$CH_3$ | H | 4 | CH | N | H | $CF_3$ | |
| 2.19 | $CH_3$ | $CH_3$ | 3-Cl | H | 4 | C-Cl | C-$CF_3$ | H | H | |
| 2.20 | $CH_3$ | $CH_3$ | H | H | 4 | CH | C-$CF_3$ | H | H | 133-135° |
| 2.21 | $CH_3$ | $CH_3$ | 2-isopropyl | H | 4 | CH | C-$CF_3$ | H | H | |
| 2.22 | $CH_3$ | $CH_3$ | H | H | 3 | CH | C-$NO_2$ | H | H | |
| 2.23 | $CH_3$ | $CH_3$ | H | H | 4 | C-Cl | C-Cl | H | H | 189-190° |
| 2.24 | $CH_3$ | $C_2H_5$ | H | H | 4 | CH | CH | $CF_3$ | H | 151-152° |
| 2.25 | $CH_3$ | $C_2H_5$ | H | H | 4 | C-Cl | C-$CF_3$ | H | H | 116-117° |
| 2.26 | $CH_3$ | $C_2H_5$ | H | H | 4 | CH | CH | $CF_3$ | Cl | 139-141° |
| 2.27 | $CH_3$ | $CH_2CH=CH_2$ | H | H | 4 | CH | CH | $CF_3$ | H | 111-113° |
| 2.28 | $CH_3$ | $CH_2CH=CH_2$ | H | H | 4 | CH | CH | $CF_3$ | Cl | 154-156° |
| 2.29 | $CH_3$ | $CH_2CH=CH_2$ | H | H | 4 | C-Cl | C-$CF_3$ | H | H | 135-137° |
| 2.30 | $CH_3$ | $CH_3$ | 2-$CH_3$ | H | 4 | CH | CH | $CF_3$ | H | |
| 2.31 | $CH_3$ | $CH_3$ | 3-$OCH_3$ | H | 4 | CH | CH | $CF_3$ | H | |
| 2.32 | $C_2H_5$ | $C_2H_5$ | 2-Cl | 6-Cl | 4 | CH | CH | $CF_3$ | H | |

Fortsetzung von Tabelle 2:

| Verb. Nr. | $R_1$ | $R_2$ | $R_4$ | $R_5$ | Position von $OR_3$ | Q | T | $R_a$ | $R_b$ | Smp [°C] |
|---|---|---|---|---|---|---|---|---|---|---|
| 2.33 | $CH_3$ | $CH_3$ | 2-$CH_3$ | H | 4 | C-Cl | C-$CF_3$ | H | H | 178-180° |
| 2.34 | $CH_3$ | $CH_3$ | 2-F | H | 4 | C-Cl | C-$CF_3$ | H | H | 152-154° |
| 2.35 | $CH_3$ | $CH_3$ | 2-$CF_3$ | H | 4 | C-Cl | C-Cl | H | H | 148-150° |
| 2.36 | $CH_3$ | $CH_3$ | 2-$C_2H_5$ | 6-$C_2H_5$ | 4 | N | CH | H | H | 112-114° |
| 2.37 | $CH_3$ | $CH_3$ | 2-$CH_3$ | 6-$CH_3$ | 4 | C-Cl | $CF_3$ | H | H | 160-162° |
| 2.38 | $CH_3$ | $CH_3$ | 3-Cl | H | 4 | N | N | $OCH_3$ | $OCH_3$ | 222-223° |
| 2.39 | $CH_3$ | $CH_3$ | H | H | 4 | N | N | $OCH_3$ | $OCH_3$ | 196-197° |
| 2.40 | $CH_3$ | $CH_3$ | 2-$CH_3$ | 6-$CH_3$ | 4 | CH | C-$CF_3$ | H | H | 168-169° |
| 2.41 | $CH_3$ | $CH_3$ | 2-$CH_3$ | 6-$CH_3$ | 4 | N | CH | H | H | 183-185° |
| 2.42 | $CH_3$ | $CH_3$ | H | H | 4 | C-$CH_3$ | C-Cl | $CH_3$ | N | |
| 2.43 | $CH_3$ | $CH_3$ | 2-$CH_3$ | 6-$CH_3$ | 4 | CH | C-Cl | H | N | |
| 2.44 | $CH_3$ | $CH_3$ | 2-Cl | 6-Cl | 4 | CH | CH | H | H | |
| 2.45 | $CH_3$ | $CH_3$ | 2-$CH_3$ | 6-$CH_3$ | 4 | C-Cl | C-Cl | H | H | |
| 2.46 | $CH_3$ | $CH_3$ | 3-Cl | 5-Cl | 4 | C-Cl | C-$CF_3$ | H | H | |
| 2.47 | $CH_3$ | $CH_3$ | H | H | 4 | C-Cl | C-$C_2Cl_5$ | H | H | |

Fortsetzung von Tabelle 2:

| Verb. Nr. | $R_1$ | $R_2$ | $R_4$ | $R_5$ | Position von $OR_3$ | Q | T | $R_a$ | $R_b$ | Smp [°C] |
|---|---|---|---|---|---|---|---|---|---|---|
| 2.48 | $CH_3$ | $CH_3$ | 3-Cl | H | 4 | C-Cl | $CCF_2CF_2Cl$ | H | H | |
| 2.49 | $CH_3$ | $CH_3$ | H | H | 4 | C-Cl | $CCF_2CF_2Cl$ | H | H | |
| 2.50 | $CH_3$ | $CH_3$ | 3-Cl | H | 4 | CH | CH | $CF_3$ | Cl | |
| 2.51 | $CH_3$ | $CH_3$ | 3-F | H | 4 | C-F | C-Cl | H | H | |
| 2.52 | $CH_3$ | $CH_3$ | 2-$C_3H_7$i | 6-$C_3H_7$i | 4 | C-Cl | $CF_3$ | H | H | |

Tabelle 3 : Pyrimidindioniumbetain-Derivate der Formel

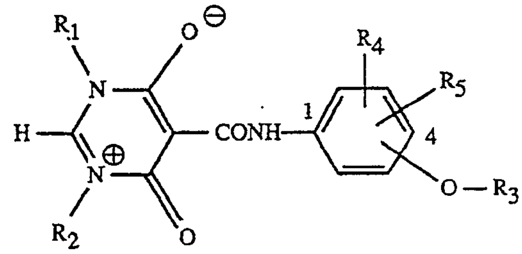

(I), worin $R_3$ folgendes bedeutet

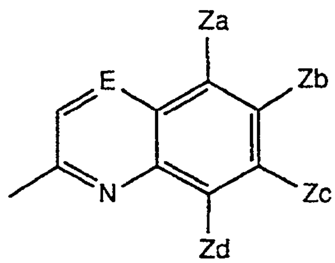

| Verb. Nr. | $R_1$ | $R_2$ | $R_4$ | $R_5$ | Position von $OR_3$ | E | Za | Zb | Zc | Zd | Smp. [°C] |
|---|---|---|---|---|---|---|---|---|---|---|---|
| 3.1 | $CH_3$ | $CH_3$ | H | H | 4 | N | H | Cl | H | H | |
| 3.2 | $CH_3$ | $CH_3$ | H | H | 4 | N | Cl | H | H | H | |
| 3.3 | $CH_3$ | $CH_3$ | H | H | 4 | N | H | F | H | H | |
| 3.4 | $CH_3$ | $CH_3$ | H | H | 4 | CH | H | H | H | H | |
| 3.5 | $CH_3$ | $CH_3$ | H | 4-Cl | 3 | N | H | H | H | H | |
| 3.6 | $CH_3$ | $CH_3$ | H | H | 4 | CH | H | Cl | H | H | |
| 3.7 | $CH_3$ | $CH_3$ | H | H | 4 | CH | H | H | Cl | Cl | |
| 3.8 | $CH_3$ | $CH_3$ | H | H | 4 | CH | H | H | H | Cl | |
| 3.9 | $CH_3$ | $CH_3$ | H | H | 4 | CH | H | $CH_3$ | H | H | |
| 3.10 | $CH_3$ | $CH_3$ | H | H | 4 | N | H | H | Cl | $CH_3$ | |
| 3.11 | $CH_3$ | $CH_3$ | H | H | 4 | N | H | H | H | $SCH_3$ | |
| 3.12 | $CH_3$ | $CH_3$ | H | H | 4 | N | $CH_3$ | H | H | Cl | |
| 3.13 | $CH_3$ | $CH_3$ | H | H | 4 | CH | $CF_3$ | H | H | H | |
| 3.14 | $CH_3$ | $CH_3$ | H | H | 4 | CH | H | $CH_3$ | H | H | |
| 3.15 | $CH_3$ | $C_2H_5$ | H | H | 4 | N | H | H | $CF_3$ | Cyclopropyl | |
| 3.16 | $CH_3$ | $CH_2CH=CH_2$ | H | H | 4 | N | H | H | $CF_3$ | Cyclopropyl | |
| 3.17 | $CH_3$ | $CH_3$ | 2-$OCH_3$ | H | 4 | N | H | H | $CF_3$ | H | |

Tabelle 4 : Pyrimidindion-Derivate der Formel

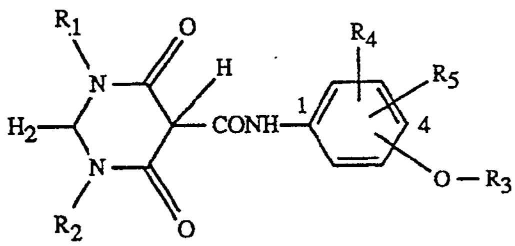

(Ia), worin $R_3$ folgendes bedeutet

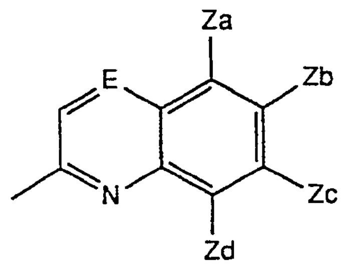

| Verb. Nr. | $R_1$ | $R_2$ | $R_4$ | $R_5$ | Position von $OR_3$ | E | Za | Zb | Zc | Zd | Smp. [°C] |
|---|---|---|---|---|---|---|---|---|---|---|---|
| 4.1 | $CH_3$ | $CH_3$ | H | H | 4 | N | H | Cl | H | H | |
| 4.2 | $CH_3$ | $CH_3$ | H | H | 4 | N | Cl | H | H | H | |
| 4.3 | $CH_3$ | $CH_3$ | H | H | 4 | N | H | F | H | H | |
| 4.4 | $CH_3$ | $CH_3$ | H | H | 4 | CH | H | H | H | H | |
| 4.5 | $CH_3$ | $CH_3$ | H | 4-Cl | 3 | N | H | H | H | H | |
| 4.6 | $CH_3$ | $CH_3$ | H | H | 4 | CH | H | Cl | H | H | |
| 4.7 | $CH_3$ | $CH_3$ | H | H | 4 | CH | H | H | Cl | Cl | |
| 4.8 | $CH_3$ | $CH_3$ | H | H | 4 | CH | H | H | H | Cl | |
| 4.9 | $CH_3$ | $CH_3$ | H | H | 4 | CH | H | $CH_3$ | H | H | |
| 4.10 | $CH_3$ | $CH_3$ | H | H | 4 | N | H | H | Cl | $CH_3$ | |
| 4.11 | $CH_3$ | $CH_3$ | H | H | 4 | N | H | H | H | $SCH_3$ | |
| 4.12 | $CH_3$ | $CH_3$ | H | H | 4 | N | $CH_3$ | H | H | Cl | |
| 4.13 | $CH_3$ | $CH_3$ | H | H | 4 | CH | $CF_3$ | H | H | H | |
| 4.14 | $CH_3$ | $CH_3$ | H | H | 4 | CH | H | $CH_3$ | H | H | |
| 4.15 | $CH_3$ | $C_2H_5$ | H | H | 4 | N | H | H | $CF_3$ | Cyclopropyl | |
| 4.16 | $CH_3$ | $CH_2CH=CH_2$ | H | H | 4 | N | H | H | $CF_3$ | Cyclopropyl | |
| 4.17 | $CH_3$ | $CH_3$ | 2-$OCH_3$ | H | 4 | N | H | H | $CF_3$ | H | |

2. Formulierungsbeispiele (%=Gewichtsprozent)

| 2.1. Emulsions-Konzentrate | a) | b) | c) |
|---|---|---|---|
| Wirkstoff aus Tabelle 1 bis 4 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus diesen Konzentraten können durch Verdünnen mit Wasser Emulsionen jeder gewünschten Konzentration hergestellt werden.

| 2.2. Emulsions-Konzentrate | a) | b) | c) |
|---|---|---|---|
| Wirkstoff aus Tabelle 1 bis 4 | 10 % | 8 % | 60 % |
| Octylphenolpolyethylenglykolether (4-5 Mol Ethylenoxid) | 3 % | 3 % | 2 % |
| Ca-Dodecylbenzolsulfonat | 3 % | 4 % | 4 % |
| Ricinusölpolyethylenglykolether (35 Mol Ethylenoxid) | 4 % | 5 % | 4 % |
| Cyclohexanon | 30 % | 40 % | 15 % |
| Xylolgemisch | 50 % | 40 % | 15 % |

Aus diesen Konzentraten können durch Verdünnen mit Wasser Emulsionen jeder gewünschten Konzentration hergestellt werden.

| 2.3. Suspensions-Konzentrat | |
|---|---|
| Wirkstoff aus Tabelle 1 bis 4 | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| 2.4. In Wasser dispergierbare Pulvermischungen | a) | b) | c) |
|---|---|---|---|
| Wirkstoff aus Tabelle 1 bis 4 | 25 % | 50 % | 75 % |
| Na-Ligninsulfonat | 5 % | 5 % | - |
| Oelsäure | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.5. Stäubemittel | a) | b) |
|---|---|---|
| Wirkstoff der Tabelle 1 bis 4 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff und Vermahlen des Gemisches erhält man gebrauchsfertige Stäubemittel.

| 2.6. Granulat | a) | b) |
|---|---|---|
| Wirkstoff der Tabelle 1 bis 4 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum eingedampft. Solche Granulate können dem Viehfutter beigemischt werden.

| 2.7. Granulat | |
|---|---|
| Wirkstoff aus Tabelle 1 bis 4 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.8. Granulat

| | |
|---|---|
| Wirkstoff aus Tabelle 1 bis 4 | 3 % |
| Polyethylenglykol (MG 200) | 3 % |
| Kaolin | 94 % |

(MG = Molekulargewicht)

Der fein gemahlene Wirkstoff wird in einem Mischer auf das mit Poly- ethylenglykol angefeuchtete Kaolin gleichmässig aufgetragen. Auf diese Weise erhält man staubfreie Umhüllungs-Granulate.

2.9. Tabletten bzw. Boli

| | | |
|---|---|---|
| I | Wirkstoff der Tabelle 1 bis 4 | 33,00 % |
| | Methylcellulose | 0,80 % |
| | Kieselsäure hochdispers | 0,80 % |
| | Maisstärke | 8,40 % |
| II | Milchzucker krist. | 22,50 % |
| | Maisstärke | 17,00 % |
| | mikrokrist. Cellulose | 16,50 % |
| | Magnesiumstearat | 1,00 % |

I Methylcellulose wird in Wasser eingerührt. Nachdem das Material gequollen ist, wird Kieselsäure eingerührt und das Gemisch homogen suspendiert. Wirkstoff und Maisstärke werden gemischt. In diese Mischung wird die wässrige Suspension eingearbeitet und zu einem Teig geknetet. Die so erhaltene Masse wird durch ein 12 M-Sieb granuliert und getrocknet.
II Alle 4 Hilfsstoffe werden gut gemischt.
III Die gemäss I und II erhaltenen Vormischungen werden gemischt und zu Tabletten oder Boli verpresst.

2.4. Injektabiles

A. Oeliges Vehikel (langsame Freisetzung)

| | |
|---|---|
| Ein Wirkstoff aus Tabelle 1 bis 4 | 0,1-1,0 g |
| Erdnussöl | ad 100 ml |
| | |
| Ein Wirkstoff aus Tabelle 1 bis 4 | 0,1-1,0 g |
| Sesamöl | ad 100 ml |

Herstellung: Der Wirkstoff wird in einem Teil des Oels unter Rühren und gegebenenfalls leichtem Erwärmen gelöst, nach Abkühlung auf das Soll- volumen aufgefüllt und durch ein geeignetes Membranfilter mit 0,22 µm sterilfiltriert.

B. Wassermischbares Lösungsmittel (mittlere Freisetzungsgeschwindigkeit)

| | |
|---|---|
| Ein Wirkstoff aus Tabelle 1 bis 4 | 0,1-1,0 g |
| 4-Hydroxymethyl-1,3-dioxolan | |
| (Glycerol Formal) | 40 g |
| 1,2-Propandiol | ad 100 ml |
| Ein Wirkstoff aus Tabelle 1 oder 2 | 0,1-1,0 g |
| Glycerindimethylketal | 40 g |
| 1,2-Propandiol | ad 100 ml |

Herstellung : Der Wirkstoff wird in einem Teil des Lösungsmittels unter Rühren gelöst, auf das Sollvolumen aufgefüllt und durch ein geeignetes Membranfilter mit 0.22 µm sterilfiltriert.

C. Wässriges Solubilisat (rasche Freisetzung)

| | |
|---|---|
| Ein Wirkstoff aus Tabelle 1 bis 4 | 0,1-1,0 g |
| Polyäthoxyliertes Ricinusöl | |
| (40 Aethylenoxideinheiten) | 10 g |
| 1,2-Propandiol | 20 g |
| Benzylalkohol | 1 g |
| Aqua ad injekt. | ad 100 ml |

| | |
|---|---|
| Ein Wirkstoff aus Tabelle 1 bis 4 | 0,1-1,0 g |
| Polyäthoxyliertes Sorbitanmonooleat | |
| (20 Aethylenoxideinheiten) | 8 g |
| 4-Hydroxymethyl-1,3-dioxolan | |
| (Glycerol Formal) | 20 g |
| Benzylalkohol | 1 g |
| Aqua ad injekt. | ad 100 ml |

Herstellung : Der Wirkstoff wird in den Lösungsmitteln und dem Tensid gelöst und mit Wasser auf das Sollvolumen aufgefüllt. Sterilfiltration durch geeignetes Membranfilter mit 0,22 µm Porendurchmesser.

Die wässrigen Systeme können bevorzugterweise auch für die orale und/oder intraruminale Applikation eingesetzt werden.

3. Biologische Beispiele

Die anthelminthische Wirksamkeit wird anhand folgender Versuche demonstriert :

3.1. Versuch an mit Nematoden wie Haemonchus contortus und Trichostrongylus colubriformis infizierten Schafen

Der Wirkstoff wird in Form einer Suspension mit einer Magensonde oder durch Pansen-Injektion Schafen verabreicht, die vorher mit Nematoden wie Haemonchus contortus und Trichostrongylus colubriformis künstlich infiziert wurden. Pro Versuch resp. pro Dosis werden 1 bis 3 Tiere verwendet. Jedes Schaf wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen wird.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich.

In diesem Test wird mit Verbindungen der Formel I eine starke Reduzierung des Nematodenbefalls erzielt. So wird beispielsweise beim Einsatz von 20 mg Wirksubstanz pro kg Körpergewicht mit den nachfolgenden Verbindungen eine Reduzierung des Nematodenbefalls von ca. 90% bewirkt: 1.1, 1.2, 1.3, 1.4, 2.1, 2.2, 2.3 und 2.4. Dieses Ergebnis wird bei einzelnen Verbindungen auch noch mit weiter herabgesetzter Dosis, beispielsweise mit 10 mg Wirksubstanz pro kg Körpergewicht oder noch geringeren Mengen an Wirksubstanz, erreicht.

3.2. Versuch an mit Cestoden wie Moniezia benedeni infizierten Schafen

Der Wirkstoff wird in Form einer Suspension mit einer Magensonde oder durch Pansen-Injektion Schafen verabreicht, die vorher mit Cestoden wie Moniezia benedeni artifiziell infiziert wurden. Pro Versuch resp. pro Dosis werden 3 Tiere verwendet. Jedes Schaf wird mit nur einer einzigen Dosis behandelt. Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich. In diesem Versuch bewirken Wirkstoffe aus der Tabelle 1 oder 2, wie beispielsweise die Verbindungen Nr. 1.1, 1.2, 1.3, 1.4, 2.1, 2.2, 2.3 oder 2.4. bei Dosen von weniger als 20 mg/kg Körpergewicht eine ca. 90%ige Reduktion des Cestodenbefalls.

3.3. Versuch an mit Fasciola hepatica infizierten Schafen

Der Wirkstoff wird in Form einer Suspension mit einer Magensonde oder durch Pansen-Injektion Schafen verabreicht, die vorher mit Fasciola hepatica artifiziell infiziert wurden. Pro Versuch resp. pro Dosis werden 3 Tiere verwendet. Jedes Tier wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen wird.

Drei bis vier Wochen nach der Behandlung werden die Schafe getötet und seziert. Die Auswertung erfolgt durch das Auszählen der nach der Behandlung in den Gallengängen zurückgebliebenen Leberegel. Gleichzeitig und gleichartig infizierte aber unbehandelte Schafe dienen als Kontrolle resp. Vergleich. Der Unterschied der in den beiden Gruppen festgestellten Anzahl von Leberegeln ergibt den Wirkungsgrad des geprüften Wirkstoffs.

In diesem Versuch zeigen Wirkstoffe aus der Tabelle 1 oder 2 bei Dosen von weniger als 20 mg Aktivsubstanz/kg Körpergewicht eine gute Wirkung gegen Fasciola hepatica. Unter diesen Wirkstoffen erweist sich die Verbindung Nr. 1.2 als besonders wirksam gegen Fasciola hepatica.

## Ansprüche

1. Verbindung der allgemeinen Formel I

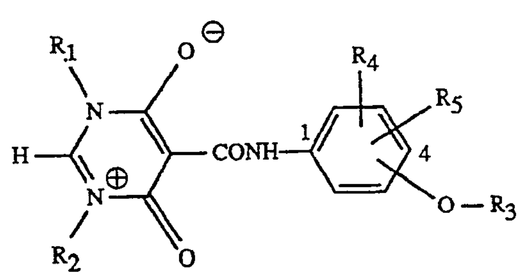

(I)

oder in ihrer hydrierten Form die Formel (Ia)

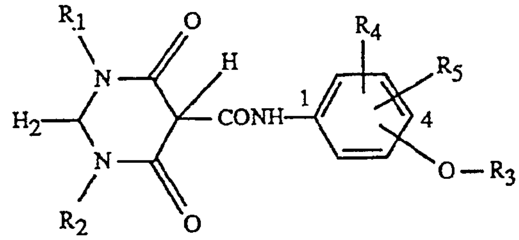

(Ia)

worin $R_1$ und $R_2$ unabhängig voneinander für $C_1$-$C_6$-Alkyl, Allyl, $C_3$-$C_6$-Cycloalkyl, Benzyl oder Phenyl stehen ;
$R_3$ einen unsubstituierten oder substituierten heteroaromatischen 6-gliederigen Ring, welcher 1, 2 oder 3 Stickstoffatome enthält oder einen benzokondensierten, unsubstituierten oder substituierten heteroaromatischen 6-gliederigen Ring, welcher 1 bis 3 Stickstoffatome enthält, und
$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl bedeuten, unter Einschluss deren tautomeren Formen und physiologisch verträglichen Salze.

2. Verbindung der Formel I nach Anspruch 1, worin $R_1$ und $R_2$ unabhängig voneinander für $C_1$-$C_6$-Alkyl, Allyl, $C_3$-$C_6$-Cycloalkyl oder Phenyl stehen und $R_3$, $R_4$ und $R_5$ die in Anspruch 1 angegebenen Bedeutungen haben.

3. Verbindung der Formel I oder Ia nach Anspruch 2, in welcher $R_1$ und $R_2$ unabhängig voneinander für $C_1$-$C_4$-Alkyl, Allyl oder Cyclopropyl stehen ; $R_3$ einen unsubstituierten oder ein- bis dreifach durch Substituenten der Gruppe $C_1$-$C_4$-Alkyl, vorzugsweise Methyl, Isopropyl und tert.-Butyl, Methoxy, Methylamino, Dimethylamino, Methylthio, Trifluormethyl, Halogen, vorzugs- weise Chlor, oder Phenyl substituierten Pyridyl, Pyrazinyl-, Pyridazinyl-, Triazinyl-, Pyrimidinyl- Chinoxalinyl-, Chinolyl- oder Chinazolinylrest, $R_4$ Wasserstoff, $C_1$-$C_4$-Alkyl, vorzugsweise Methyl oder Isopropyl, oder Methoxy, und $R_5$ Wasserstoff oder Methyl bedeuten.

4. Verbindung der Formel I oder Ia nach Anspruch 2, in welcher $R_1$ $C_1$-$C_4$-Alkyl, Allyl oder $C_3$-$C_6$-Cycloalkyl, $R_2$ $C_1$-$C_4$-Alkyl, $R_3$ einen unsubstituierten oder ein- bis dreifach durch Substituenten der Gruppe $C_1$-$C_4$-Alkyl, Halogen-$C_1$-$C_4$-Alkyl, $C_1$-$C_4$-Alkoxy, Halogen-$C_1$-$C_4$-Alkoxy, $C_1$-$C_4$-Alkylthio, $C_1$-$C_3$-Alkylamino, Di-($C_1$-$C_3$-alkyl)-amino, Allyl, Halogen, $C_3$-$C_6$-Cycloalkyl oder Phenyl substituierten, über ein Kohlenstoffatom gebundenen Pyridyl, Pyrazinyl-, Pyrimidinyl-, Pyridazinyl-, Triazinyl-, Chinoxalinyl- oder Chinolinylrest, $R_4$ Wasserstoff, $C_1$-$C_4$-Alkyl oder $C_1$-$C_4$-Alkoxy, und $R_5$ Wasserstoff bedeuten.

5. Verbindung der Formel I oder Ia nach Anspruch 4, in welcher $R_1$ $C_1$-$C_2$-Alkyl, Allyl, oder Cyclopropyl, $R_2$ $C_1$-$C_2$-Alkyl, $R_3$ einen unsubstituierten oder ein- bis dreifach durch Substituenten der Gruppe $C_1$-$C_4$-Alkyl, Halogen-$C_1$-$C_4$-Alkyl, $C_1$-$C_4$-Alkoxy, $C_1$-$C_4$-Alkylthio, Di-($C_1$-$C_3$-alkyl)-amino, Halogen oder Phenyl substituierten, über ein Kohlenstoffatom gebundenen Pyridyl, Pyrazinyl-, Pyrimidinyl-, Pyridazinyl-, Triazinyl-, Chinoxalinyl-oder Chinolinylrest, $R_4$ Wasserstoff, $C_1$-$C_4$-Alkyl oder $C_1$-$C_4$-Alkoxy, und $R_5$ Wasserstoff oder $C_1$-$C_4$-Alkyl bedeuten.

6. Verbindung der Formel I oder Ia nach Anspruch 5, in welcher $R_1$ $C_1$-$C_2$-Alkyl, Allyl oder Cyclopropyl, $R_2$ Methyl, $R_3$ einen unsubstituierten oder ein- bis dreifach durch Substituenten der Gruppe $C_1$-$C_4$-Alkyl, Trifluormethyl, Methoxy, Methylthio, Chlor oder Phenyl substituierten, über ein Kohlenstoffatom gebundenen Pyridyl, Pyrimidinyl-, Pyrazinyl- oder Pyridazinylrest, $R_4$ Wasserstoff, $C_1$-$C_4$-Alkyl oder $C_1$-$C_4$-Alkoxy, und $R_5$ Wasserstoff oder $C_1$-$C_4$-Alkyl bedeuten und der Molekülteil —$OR_3$ in meta- oder para-Stellung zum Stickstoffatom der Carbamoylgruppe steht.

7. Verbindung der Formel I oder Ia nach Anspruch 6, in welcher $R_1$ Methyl oder Allyl, $R_2$ Methyl, $R_3$ einen unsubstituierten oder ein- oder zweifach durch Substituenten der Gruppe $C_1$-$C_2$-Alkyl, Halogen-$C_1$-$C_2$-Alkyl, Halogen oder Phenyl substituierten, über ein Kohlenstoffatom gebundenen Pyridyl- oder Pyrimidinylring, $R_4$ Wasserstoff, $C_1$-$C_2$-Alkyl oder $C_1$-$C_2$-Alkoxy, $R_5$ Wasserstoff oder Methyl bedeuten und der Molekülteil —$OR_3$ in meta- oder para-Stellung zum Stickstoffatom der Carbamoylgruppe steht.

8. Verbindung der Formel I oder Ia nach Anspruch 7, in welcher $R_1$ Methyl, $R_2$ Methyl, $R_3$ einen ein- oder zweifach durch Substituenten der Gruppe Methyl, Trifluormethyl, Chlor oder Phenyl substituierten, über

ein Kohlenstoffatom gebundenen Pyridyl- oder Pyrimidinylring, $R_4$ Wasserstoff, Methyl, Isopropyl, Methoxy oder Ethoxy, $R_5$ Wasserstoff oder Methyl bedeuten und der Molekülteil —$OR_3$ in meta- oder para-Stellung zum Stickstoffatom der Carbamoylgruppe steht.

**9.** Verbindung der Formel I oder Ia nach Anspruch 8, in welcher $R_1$ Methyl, $R_2$ Methyl, $R_3$ einen durch Trifluormethyl einfach oder durch Trifluormethyl und einen weiteren Substituenten der Gruppe Methyl, Trifluormethyl, Chlor und Phenyl insgesamt zweifach substituierten, über ein Kohlenstoffatom gebundenen Pyridyl- oder Pyrimidinylring, $R_4$ Wasserstoff, Methyl, Isopropyl oder Methoxy, $R_5$ Wasserstoff oder Methyl bedeuten und der Molekülteil —$OR_3$ in para-Stellung zum Stickstoffatom der Carbamoylgruppe steht.

**10.** Eine Verbindung der Formel I nach Anspruch 2, ausgewählt aus der Reihe :
1,3-Dimethyl-5-[4-(4-trifluormethyl-6-chlorpyridyl-2-oxy)-phenylcarbamoyl]-4(6)-oxo-6(4)-oxido-(1H, 5H)-pyrimidiniumbetain ;
1,3-Dimethyl-5-[4-(2-cyclopropyl-6-trifluormethylpyrimidyl-4-oxy)-phenylcarbamoyl]-4(6)-oxo-6(4)-oxido-(1H,5H)-pyrimidiniumbetain ;
1,3-Dimethyl-3-[3-(3-chlor-5-trifluormethylpyridyl-2-oxy)-4-chlorphenylcarbamoyl]-4(6)-oxo-6(4)-oxido-(1H,5H)-pyrimidiniumbetain ;
1,3-Dimethyl-5-[4-(4-trifluormethylpyridyl-2-oxy)-phenylcarbamoyl]-4(6)-oxo-6(4)-oxido-(1H,5H)-pyrimidiniumbetain ;
1,3-Dimethyl-5-[4-(3-chlor-5-trifluormethylpyridyl-2-oxy)-phenylcarbamoyl]-4(6)-oxo-6(4)-oxido-(1H, 5H)-pyrimidiniumbetain.

**11.** Eine Verbindung der Formel Ia nach Anspruch 2, ausgewählt aus der Reihe :
1,3-Dimethyl-5-[4-(4-trifluormethyl-6-chlorpyridyl-2-oxy)-phenylcarbamoyl]-4,6-(1H,3H,5H)-pyrimidindion ;
1,3-Dimethyl-5-[4-(2-cyclopropyl-6-trifluormethylpyrimidyl-4-oxy)-phenylcarbamoyl]-4,6-(1H,3H,5H)-pyrimidindion ;
1,3-Dimethyl-3-[3-(3-chlor-5-trifluormethylpyridyl-2-oxy)-4-chlorphenylcarbamoyl]-4,6-(1H,3H,5H)-pyrimidindion ;
1,3-Dimethyl-5-[4-(4-trifluormethylpyridyl-2-oxy)-phenylcarbamoyl]-4,6-(1H,3H,5H)-pyrimidindion ;
1,3-Dimethyl-5-[4-(3-chlor-5-trifluormethylpyridyl-2-oxy)-phenylcarbamoyl]-4,6-(1H,3H,5H)-pyrimidindion.

**12.** Verfahren zur Herstellung einer Verbindung der Formel I

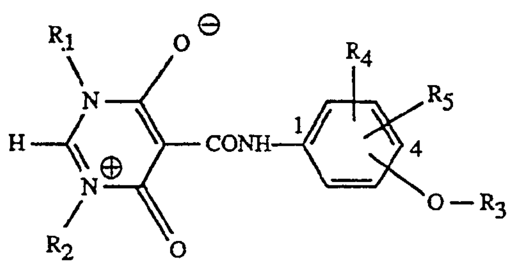

(I)

oder in ihrer hydrierten Form die Formel (Ia)

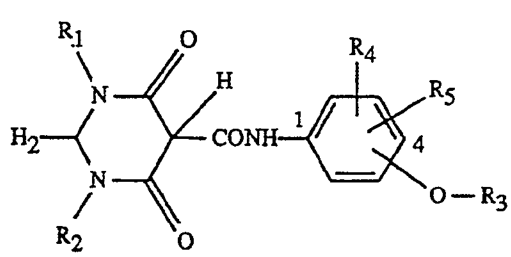

(Ia)

worin $R_1$ und $R_2$ unabhängig voneinander für $C_1$-$C_6$-Alkyl, Allyl, $C_3$-$C_6$-Cycloalkyl, Benzyl oder Phenyl stehen ;
$R_3$ einen unsubstituierten oder substituierten heteroaromatischen 6-gliederigen Ring, welcher 1, 2 oder 3 Stickstoffatome enthält oder einen benzokondensierten, unsubstituierten oder substituierten heteroaromatischen 6-gliederigen Ring, welcher 1 bis 3 Stickstoffatome enthält, und
$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl bedeuten, unter Einschluss deren tautomeren Formen und physiologisch verträglichen Salze, dadurch gekennzeichnet, dass man eine Verbindung der Formel II,

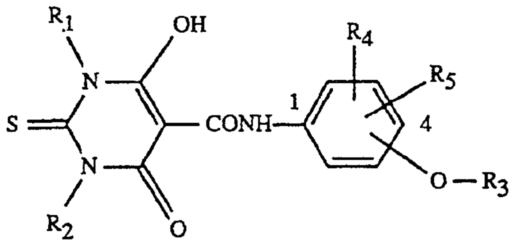

(II)

worin $R_1$, $R_2$ und $R_3$ die unter Formel I und Ia angegebenen Bedeutungen haben entschwefelt.

13. Verfahren nach Anspruch 12, dadurch gekennzeichnet, dass man die Entschwefelung durch katalytische Hydrierung in einem reaktionsinerten Lösungsmittel oder Lösungsmittelgemisch bei Temperaturen in einem Bereich von 20 bis 180°C, vorzugsweise 60 bis 120°C, insbesondere bei Rückflusstemperatur vornimmt.

14. Verfahren nach Anspruch 13, dadurch gekennzeichnet, dass man die Hydrierung mit einem Trialkylzinnhydrid, Trialkylzinnhalogenid, Trialkylgermaniumhydrid, Trialkylgermaniumhalogenid, Alkylquecksilberhydrid oder Alkylquecksilberhalogenid durchführt und in den Fällen in denen man ein Halogenid einsetzt, zusätzlich in Gegenwart von $NaBH_4$ arbeitet.

15. Verfahren nach Anspruch 14, dadurch gekennzeichnet, dass man in Gegenwart eines Radikalinitiators arbeitet.

16. Anthelmintisches Mittel, dadurch gekennzeichnet, dass es als aktive Komponente mindestens eine Verbindung der Formel I oder Ia, ein Tautomeres oder ein Salz davon gemäss Anspruch 1 bis 11 neben Träger- und weiteren Hilfsstoffen enthält.

17. Verbindung der Formel I oder Ia nach einem der Ansprüche 1 bis 11 zur Verwendung in einem Verfahren zur Bekämpfunhg von Wurmerkrankungen bei Haus- und Nutztieren.

18. Verbindung der Formel I oder Ia nach einem der Ansprüche 1 bis 11 zur Verwendung zur Herstellung eines veterinärmedizinischen Pharmazeutikums.

**Ansprüche für folgenden Vertragsstaaten : GR und ES**

1. Verfahren zur Herstellung eines Wirkstoffs der Formel I

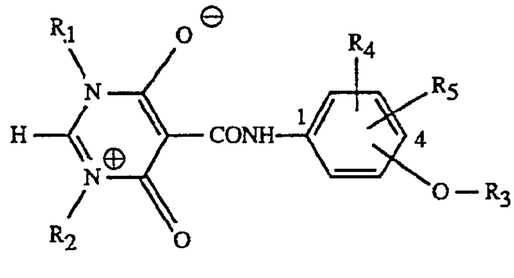

(I)

oder in ihrer hydrierten Form die Formel (Ia)

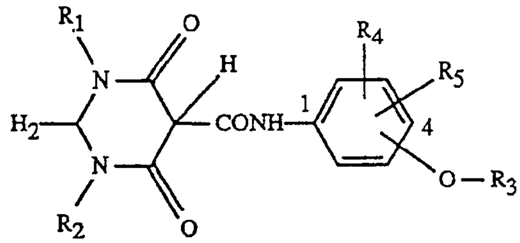

(Ia)

worin $R_1$ und $R_2$ unabhängig voneinander für $C_1$-$C_6$-Alkyl, Allyl, $C_3$-$C_6$-Cycloalkyl, Benzyl oder Phenyl stehen ;
$R_3$ einen unsubstituierten oder substituierten heteroaromatischen 6-gliederigen Ring, welcher 1, 2 oder 3 Stickstoffatome enthält oder einen benzokondensierten, unsubstituierten oder substituierten heteroaromatischen 6-gliederigen Ring, welcher 1 bis 3 Stickstoffatome enthält, und
$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl bedeuten, unter Einschluss deren tautomeren Formen und physiologisch verträglichen Salze, dadurch gekennzeichnet, dass man eine Verbindung der Formel II,

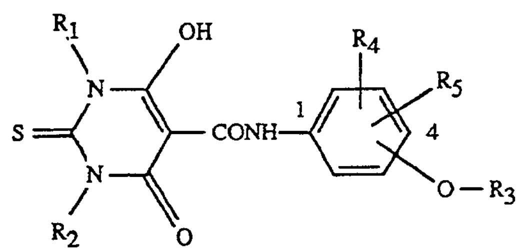

(II)

worin $R_1$, $R_2$ und $R_3$ die unter Formel I und Ia angegebenen Bedeutungen haben entschwefelt.

2. Verfahren nach Anspruch 1, dadurch gekennzeichnet, dass man die Entschwefelung durch katalytische Hydrierung in einem reaktionsinerten Lösungsmittel oder Lösungsmittelgemisch bei Temperaturen in einem Bereich von 20 bis 180°C, vorzugsweise 60 bis 120°C, insbesondere bei Rückflusstemperatur vornimmt.

3. Verfahren nach Anspruch 2, dadurch gekennzeichnet, dass man die Hydrierung mit einem Trialkylzinnhydrid, Trialkylzinnhalogenid, Trialkylgermaniumhydrid, Trialkylgermaniumhalogenid, Alkylquecksilberhydrid oder Alkylquecksilberhalogenid durchführt und in den Fällen in denen man ein Halogenid einsetzt, zusätzlich in Gegenwart von $NaBH_4$ arbeitet.

4. Verfahren nach Anspruch 3, dadurch gekennzeichnet, dass man in Gegenwart eines Radikalinitiators arbeitet.

5. Verfahren nach einem der Ansprüche 1 bis 4, dadurch gekennzeichnet, dass es sich bei dem Wirkstoff um eine Verbindung der Formel I oder Ia handelt, worin $R_1$ und $R_2$ unabhängig voneinander für $C_1$-$C_6$-Alkyl, Allyl, $C_3$-$C_6$-Cycloalkyl oder Phenyl stehen und $R_3$, $R_4$ und $R_5$ die in Anspruch 1 angegebenen Bedeutungen haben.

6. Verfahren nach Anspruch 5, dadurch gekennzeichnet, dass es sich bei dem Wirkstoff um eine Verbindung der Formel I oder Ia handelt, in welcher $R_1$ und $R_2$ unabhängig voneinander für $C_1$-$C_4$-Alkyl, Allyl oder Cyclopropyl stehen ; $R_3$ einen unsubstituierten oder ein- bis dreifach durch Substituenten der Gruppe $C_1$-$C_4$-Alkyl, vorzugsweise Methyl, Isopropyl und tert.-Butyl, Methoxy, Methyl- amino, Dimethylamino, Methylthio, Trifluormethyl, Halogen, vorzugs- weise Chlor, oder Phenyl substituierten Pyridyl, Pyrazinyl-, Pyridazinyl-, Triazinyl-, Pyrimidinyl- Chinoxalinyl-, Chinolyl- oder Chinazolinylrest, $R_4$ Wasserstoff, $C_1$-$C_4$-Alkyl, vorzugsweise Methyl oder Isopropyl, oder Methoxy, und $R_5$ Wasserstoff oder Methyl bedeuten.

7. Verfahren nach Anspruch 5, dadurch gekennzeichnet, dass es sich bei dem Wirkstoff um eine Verbindung der Formel I oder Ia handelt, in welcher $R_1$ $C_1$-$C_4$-Alkyl, Allyl oder $C_3$-$C_6$-Cycloalkyl, $R_2$ $C_1$-$C_4$-Alkyl, $R_3$ einen unsubstituierten oder ein- bis dreifach durch Substituenten der Gruppe $C_1$-$C_4$-Alkyl, Halogen-$C_1$-$C_4$-Alkyl,

$C_1$-$C_4$-Alkoxy, Halogen-$C_1$-$C_4$-Alkoxy, $C_1$-$C_4$-Alkylthio, $C_1$-$C_3$-Alkylamino, Di-($C_1$-$C_3$-alkyl)-amino, Allyl, Halogen, $C_3$-$C_6$-Cycloalkyl oder Phenyl substituierten, über ein Kohlenstoffatom gebundenen Pyridyl, Pyrazinyl-, Pyrimidinyl-, Pyridazinyl-, Triazinyl-, Chinoxalinyl- oder Chinolinylrest, $R_4$ Wasserstoff, $C_1$-$C_4$-Alkyl oder $C_1$-$C_4$-Alkoxy, und $R_5$ Wasserstoff bedeuten.

8. Verfahren nach Anspruch 7, dadurch gekennzeichnet, dass es sich bei dem Wirkstoff um eine Verbindung der Formel I oder Ia handelt, in welcher $R_1$ $C_1$-$C_2$-Alkyl, Allyl, oder Cyclopropyl, $R_2$ $C_1$-$C_2$-Alkyl, $R_3$ einen unsubstituierten oder ein- bis dreifach durch Substituenten der Gruppe $C_1$-$C_4$-Alkyl, Halogen-$C_1$-$C_4$-Alkyl, $C_1$-$C_4$-Alkoxy, $C_1$-$C_4$-Alkylthio, Di-($C_1$-$C_3$-alkyl)-amino, Halogen oder Phenyl substituierten, über ein Kohlenstoffatom gebundenen Pyridyl, Pyrazinyl-, Pyrimidinyl-, Pyridazinyl-, Triazinyl-, Chinoxalinyl-oder Chinolinylrest, $R_4$ Wasserstoff, $C_1$-$C_4$-Alkyl oder $C_1$-$C_4$-Alkoxy, und $R_5$ Wasserstoff oder $C_1$-$C_4$-Alkyl bedeuten.

9. Verfahren nach Anspruch 8, dadurch gekennzeichnet, dass es sich bei dem Wirkstoff um eine Verbindung der Formel I oder Ia handelt, in welcher $R_1$ $C_1$-$C_2$-Alkyl, Allyl oder Cyclopropyl, $R_2$ Methyl, $R_3$ einen unsubstituierten oder ein- bis dreifach durch Substituenten der Gruppe $C_1$-$C_4$-Alkyl, Trifluormethyl, Methoxy, Methylthio, Chlor oder Phenyl substituierten, über ein Kohlenstoffatom gebundenen Pyridyl, Pyrimidinyl-, Pyrazinyl- oder Pyridazinylrest, $R_4$ Wasserstoff, $C_1$-$C_4$-Alkyl oder $C_1$-$C_4$-Alkoxy, und $R_5$ Wasserstoff oder $C_1$-$C_4$-Alkyl bedeuten und der Molekülteil —$OR_3$ in meta- oder para-Stellung zum Stickstoffatom der Carbamoylgruppe steht.

10. Verfahren nach Anspruch 9, dadurch gekennzeichnet, dass es sich bei dem Wirkstoff um eine Verbindung der Formel I oder Ia handelt, in welcher $R_1$ Methyl oder Allyl, $R_2$ Methyl, $R_3$ einen unsubstituierten oder ein- oder zweifach durch Substituenten der Gruppe $C_1$-$C_2$-Alkyl, Halogen-$C_1$-$C_2$-Alkyl, Halogen oder Phenyl substituierten, über ein Kohlenstoffatom gebundenen Pyridyl- oder Pyrimidinylring, $R_4$ Wasserstoff, $C_1$-$C_2$-Alkyl oder $C_1$-$C_2$-Alkoxy, $R_5$ Wasserstoff oder Methyl bedeuten und der Molekülteil —$OR_3$ in meta- oder para-Stellung zum Stickstoffatom der Carbamoylgruppe steht.

11. Verfahren nach Anspruch 10, dadurch gekennzeichnet, dass es sich bei dem Wirkstoff um eine Verbindung der Formel I oder Ia handelt, in welcher $R_1$ Methyl, $R_2$ Methyl, $R_3$ einen ein- oder zweifach durch Substituenten der Gruppe Methyl, Trifluormethyl, Chlor oder Phenyl substituierten, über ein Kohlenstoffatom gebundenen Pyridyl- oder Pyrimidinylring, $R_4$ Wasserstoff, Methyl, Isopropyl, Methoxy oder Ethoxy, $R_5$ Wasserstoff oder Methyl bedeuten und der Molekülteil —$OR_3$ in meta- oder para-Stellung zum Stickstoffatom der Carbamoylgruppe steht.

12. Verfahren nach Anspruch 11, dadurch gekennzeichnet, dass es sich bei dem Wirkstoff um eine Verbindung der Formel I oder Ia handelt, in welcher $R_1$ Methyl, $R_2$ Methyl, $R_3$ einen durch Trifluormethyl einfach oder durch Trifluormethyl und einen weiteren Substituenten der Gruppe Methyl, Trifluormethyl, Chlor und Phenyl insgesamt zweifach substituierten, über ein Kohlenstoffatom gebundenen Pyridyl- oder Pyrimidinylring, $R_4$ Wasserstoff, Methyl, Isopropyl oder Methoxy, $R_5$ Wasserstoff oder Methyl bedeuten und der Molekülteil —$OR_3$ in para-Stellung zum Stickstoffatom der Carbamoylgruppe steht.

13. Verfahren nach Anspruch 5, dadurch gekennzeichnet, dass es sich bei dem Wirkstoff um eine Verbindung der Formel I handelt, ausgewählt aus der Reihe :

1,3-Dimethyl-5-[4-(4-trifluormethyl-6-chlorpyridyl-2-oxy)-phenylcarbamoyl]-4(6)-oxo-6(4)-oxido-(1H,5H)-pyrimidiniumbetain ;

1,3-Dimethyl-5-[4-(2-cyclopropyl-6-trifluormethylpyrimidyl-4-oxy)-phenylcarbamoyl]-4(6)-oxo-6(4)-oxido-(1H,5H)-pyrimidiniumbetain ;

1,3-Dimethyl-3-[3-(3-chlor-5-trifluormethylpyridyl-2-oxy)-4-chlorphenylcarbamoyl]-4(6)-oxo-6(4)-oxido-(1H,5H)-pyrimidiniumbetain ;

1,3-Dimethyl-5-[4-(4-trifluormethylpyridyl-2-oxy)-phenylcarbamoyl]-4(6)-oxo-6(4)-oxido-(1H,5H)-pyrimidiniumbetain ;

1,3-Dimethyl-5-[4-(3-chlor-5-trifluormethylpyridyl-2-oxy)-phenylcarbamoyl]-4(6)-oxo-6(4)-oxido-(1H,5H)-pyrimidiniumbetain.

14. Verfahren nach Anspruch 5, dadurch gekennzeichnet, dass es sich bei dem Wirkstoff um eine Verbindung der Formel Ia handelt, ausgewählt aus der Reihe :

1,3-Dimethyl-5-[4-(4-trifluormethyl-6-chlorpyridyl-2-oxy)-phenylcarbamoyl]-4,6-(1H,3H,5H)-pyrimidin

dion ;
1,3-Dimethyl-5-[4-(2-cyclopropyl-6-trifluormethylpyrimidyl-4-oxy)-phenylcarbamoyl]-4,6-(1H,3H,5H)-pyrimidindion ;
1,3-Dimethyl-3-[3-(3-chlor-5-trifluormethylpyridyl-2-oxy)-4-chlorphenylcarbamoyl]-4,6-(1H,3H,5H)-pyrimidindion ;
1,3-Dimethyl-5-[4-(4-trifluormethylpyridyl-2-oxy)-phenylcarbamoyl]-4,6-(1H,3H,5H)-pyrimidindion ;
1,3-Dimethyl-5-[4-(3-chlor-5-trifluormethylpyridyl-2-oxy)-phenylcarbamoyl]-4,6-(1H,3H,5H)-pyrimidindion.

**15.** Verfahren zur Bekämpfung parasitärer Helminthen, dadurch gekennzeichnet, dass man einem Tier eine anthelmintisch wirksame Menge einer Verbindung der Formel I oder Ia gemäss Anspruch 5 bis 14 verabreicht.

**16.** Verwendung einer Verbindung der Formel I oder Ia gemäss einem der Ansprüche 5 bis 14 zur Bekämpfung von parasitären Helminthen.